# EUROPEAN PATENT APPLICATION

(11) **EP 2 957 634 A1**
(43) Date of publication of application: **23.12.2015**
(21) Application number: 14382239.3
(22) Date of filing: 20.06.2014
(51) Int. Cl.: C12N 15/113, A61K 31/713, A61P 11/00, A61P 43/00, C12Q 1/68, G01N 33/50

(54) **Compounds for prevention and/or treatment of fibrotic diseases**

(71) Applicant: Consejo Superior De Investigaciones Científicas, 28006 Madrid (ES); Gendiag.exe, S.L., 08950 Esplugues de Llobregat (Barcelona) (ES)
(72) Inventor: Lamas Peláez, Santiago, E-28006 Madrid (ES); Fierro Fernández, Marta, E-28006 Madrid (ES)
(74) Representative: ABG Patentes, S.L.

(57) **Abstract**

The present invention relates to compounds for their use in the prevention and/or treatment of a fibrotic disease in a subject, in particular to agents selected from the group consisting of miR-9, a variant thereof, an agent inducing the expression of miR-9 and an agent inducing the expression of a variant of miR-9, as well as to a diagnostic method of a fibrotic disease in a subject based on the miR-9 expression level in a sample from said subject.

SEQUENCE LISTING

<110> CONSEJO SUPERIOR DE INVESTIGACIONES CIENTÍFICAS
GENDIAG.EXE, S.L.

<120> COMPOUNDS FOR PREVENTION AND/OR TREATMENT OF FIBROTIC DISEASES

<130> P10682EP00

<160> 5

<170> PatentIn version 3.5

<210> 1
<211> 23
<212> RNA
<213> Homo sapiens

<400> 1
ucuuugguua ucuagcugua uga 23

<210> 2
<211> 89
<212> RNA
<213> Homo sapiens

<400> 2
cgggguuggu uguuaucuuu gguuaucuag cuguaugagu gguguggagu cuucauaaag 60

cuagauaacc gaaaguaaaa auaacccca 89

<210> 3
<211> 87
<212> RNA
<213> Homo sapiens

<400> 3
ggaagcgagu uguuaucuuu gguuaucuag cuguaugagu guauuggucu ucauaaagcu 60

agauaaccga aaguaaaaac uccuuca 87

<210> 4
<211> 90
<212> RNA
<213> Homo sapiens

<400> 4
ggaggcccgu uucucucuuu gguuaucuag cuguaugagu gccacagagc cgucauaaag 60

cuagauaacc gaaaguagaa augauucuca 90

<210> 5
<211> 89
<212> RNA
<213> Mus musculus

<400> 5
cgggguuggu uguuaucuuu gguuaucuag cuguaugagu gguguggagu cuucauaaag 60

cuagauaacc gaaaguaaaa auaacccca 89

## Description

### FIELD OF THE INVENTION

The present invention relates to compounds for their use in the prevention and/or treatment of a fibrotic disease in a subject, in particular to agents selected from the group consisting of miR-9 and an agent inducing the expression of miR-9, as well as to a diagnostic method of a fibrotic disease in a subject that comprises determining the miR-9 expression level in a sample from said subject.

### BACKGROUND OF THE INVENTION

Idiopathic pulmonary disease (IPF) is a chronic disease causing inflammation and fibrosis (scarring) of tissue in the lungs. There are various types with similar symptoms, but different response to treatment and outcomes. Although its cause is unknown, researchers believed that IPF is an exaggerated and uncontrolled inflammatory response producing the scar tissue. Over time, scarring surrounds the thin walled air sacs in the lungs, making the tissue thicker and stiffer. As a result, breathing becomes difficult and the lungs gradually lose their ability to pass oxygen to the rest of the body. IPF risk factors include age (over 50); cigarette smoking; viral infection; occupational exposures to dusts containing wood, metal, silica, bacteria, and animal proteins or to aerosol sprays, gases and fumes; medications such as nitrofurantoin, sulfasalazine, amiodarone, propranolol, methotrexate, cyclophosphamide or bleomycin; gastroesophageal reflux disease (GERD), and other family members with IPF.

Although the efforts made to date, there is still no cure for IPF. Current treatments are aimed at improving symptoms and slowing the disease process by reducing inflammation and scarring. However, the tissue that is already scarred cannot be returned to normal. Current treatments include the following
- combination of prednisone to reduce inflammation and cytoxan or azathioprine to reduce the body's immune response, and
- agents slowing the progression of the fibrosis: high dose n-acetylcysteine, pirfenidone, interferon-gamma 1b, coumadin, etanercept, bosentan, imatinib, sildenafil, pirfenidone, colchicine, methotrexate, penicillamine and cyclosporine.

Methods for the treatment of idiopathic pulmonary fibrosis (IPF) based on an antibody anti-connective tissue growth factor (anti-CTGF) have been described in WO 2013165590 A1. Methods for the treatment of IPF based on a liposomally formulated reduced glutathione have been described in US 20140023696 A1.

Methods for the prognosis, diagnosis and treatment of IPF based on the expression levels of a set of genes have been described in WO 2013148232 A1. Similarly, methods for the diagnosis of idiopathic pulmonary fibrosis based on serum marker levels have been described in US 20120035067 A1.

However, idiopathic pulmonary fibrosis (IPF), a progressive fibrotic interstitial lung disease (ILD) with median survival of 2.5-3 years, is still largely unaffected by currently available medical therapies. Therefore, in view of the state of the art, there is still a need to develop strategies to reduce and/or to prevent damages caused by IPF, as well as fibrosis occurring in organs others than lungs.

### SUMMARY OF THE INVENTION

The authors of the present invention have found that both oxidative stress and TGF-β1 induce miR-9 expression in pulmonary fibroblasts. Increasing miR-9 levels significantly reduced TGFBRII and TGF-β1-NOX4 induced expression in pulmonary fibroblasts. This effect correlated with a decrease in TGF-β1-mediated signaling. Up-regulation of miR-9 levels attenuated TGF-β-induced transformation of lung fibroblasts into myofibroblasts (see Example 1). These contractile fibroblasts are believed to be the main source of abnormal extracellular matrix production in organ fibrosis. Thus, the authors concluded that miR-9 down-regulates the profibrogenic activities of TGF-β1. Assays performed in a murine model of lung fibrosis confirmed that the increase of miR-9 expression levels prevented bleomycin-induced lung fibrosis, as well as that miR-9 targets NOX4 and TGFBR2 (see Example 2).

The inventors have also found that miR-9 plays a significant role in the TGF-β1 mediated transformation of mesenchymal cells into fibrogenic myofibroblasts, indicating a protective role for miR-9 in the initiation and progression of peritoneal fibrosis (see Example 4). Similarly, they have confirmed TGF-β1-induced miR-9 expression also in human dermal fibroblasts, as well as that miR-9 plays a significant role in the TGF-β1 mediated transformation of fibroblasts into fibrogenic myofibroblasts in skin fibrosis (see Example 5).

Therefore, the invention relates to an agent selected from the group consisting of miR-9, a miR-9 variant, an agent inducing the expression of miR-9 and an agent inducing the expression of a variant of miR-9 for use in the prevention and/or treatment of a fibrotic disease in a subject.

Additionally, the inventors have found that lung samples from patients suffering from idiopathic pulmonary fibrosis showed increased miR-9 expression levels in comparison to control subjects, suggesting a role for miR-9 expression levels in the diagnosis of a fibrotic disease (see Example 3).

Thus, in a further aspect, the invention relates to a method for diagnosing a fibrotic disease in a subject comprising
(i) determining the level of expression of miR-9 in a sample from said subject, and
(ii) comparing the level of expression in (i) to a reference value,
wherein if the expression level of miR-9 is increased with respect to the reference value, then the subject is diagnosed with a fibrotic disease.

### BRIEF DESCRIPTION OF THE FIGURES

**Figure 1****.** (A) H₂O₂ induces miR-9 expression in human lung fibroblasts (HFL-1). (B) TGF-β1 induces ROS production, measured by flow cytometry using DCHF-DA probe (DCF), and (C) up-regulates miR-9 expression in HFL-1.
**Figure 2****.** miR-9 binds directly to the human 3'-UTR of (A) NOX4 and (B) TGFBRII genes, detected by a decrease in the luminiscence of a luciferase reporter plasmid. Increasing miR-9 levels by transfection of miR-9 precursors decreased TGFBRII- and TGF-β1-NOX4-induced expression, at the mRNA (B and E, respectively) and protein levels (C and F, respectively).
**Figure 3****.** Increasing miR-9 levels in pulmonary fibroblasts delays TGF-β1-induced transcription of (A) α-smooth muscle actin (α-SMA), (B) collagen 1α1 (Col 1α1) and (C) fibronectin (FN). Similarly, up-regulation of miR-9 decreases (D) α-SMA and (E) FN protein level.
**Figure 4****.** Increasing miR-9 levels reduced Smad2/3 signaling, reflected in (A) the decrease in the luminiscence of the CAGAluc luciferase reporter plasmid, (B) increased Smad7 expression, and (C) attenuated Smad2 phosphorylation in response to TGF-β1 stimulation in pulmonary fibroblasts.

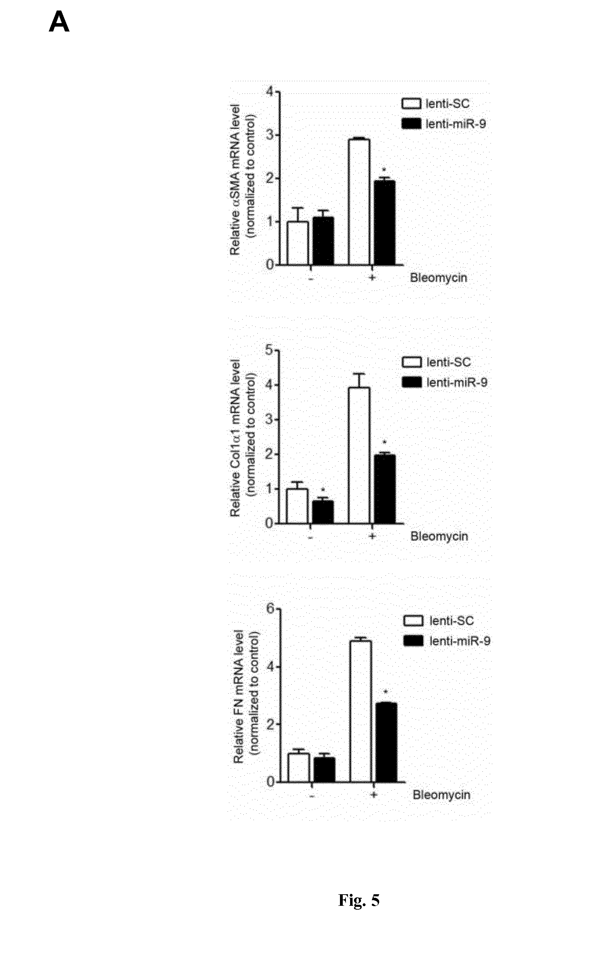

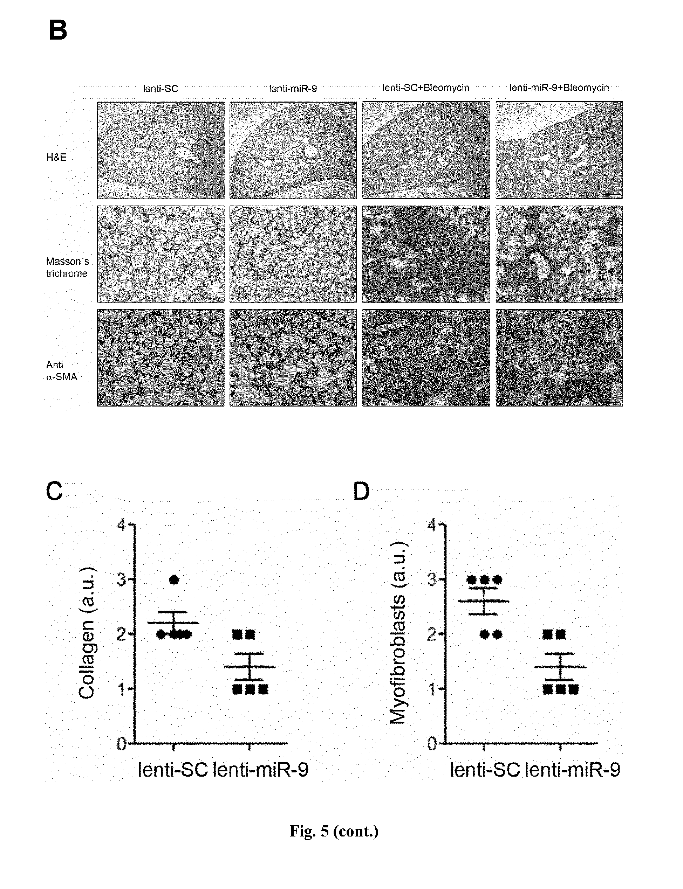

**Figure 5****.** (A) mRNA levels of α-SMA, Col 1α1 and FN in mice receiving lentimiR scramble (lenti-SC), mice receiving lentimiR-9 (lenti-miR-9), mice receiving lentimiR scramble and administered with bleomycin (lenti-SC + bleomycin) and mice receiving lentimiR-9 and administered with bleomycin (lenti-miR-9 + bleomycin) (n=4 in each group). Data represent the mean±SEM, *p<0.05 compared with bleomycin-treated mice with lenti-SC. (B) Pulmonary fibrosis was assessed by hematoxylin/eosin staining and Masson's trichrome blue staining for collagen, and by inmunohistochemistry using an anti-α-SMA antibody for α-SMA. (C) Collagen cuantification. (D) Myofibriblast cuantification.
**Figure 6****.** (A) mRNA levels of NOX4 in mice receiving lentimiR scramble (lenti-SC), mice receiving lentimiR-9 (lenti-miR-9), mice receiving lentimiR scramble and administered with bleomycin (lenti-SC + bleomycin) and mice receiving lentimiR-9 and administered with bleomycin (lenti-miR-9 + bleomycin) (n=4 in each group). Data represent the mean±SEM, *p<0.05 compared with bleomycin-treated mice given lenti-SC. (B) mRNA levels of TFBR2 in groups as above. Data represent the mean±SEM, *p<0.05 compared with mice given lenti-SC.
**Figure 7****.** (A) mRNA levels of miR-9 in mice being administered with bleomycin oropharingeally. n=4 in each group, data represent the mean±SEM, *p<0.05 compared to day 0. (B) *In situ* hybridization to localize miR-21 performed in lung sections prepared from mouse lungs harvested at day 0 and day 14 after bleomycin instillation. (C) miR-9 levels in histologically normal lungs and in lungs from patients with IPF. Data represent the mean ± SEM, *p<0.05 compared with control normal lungs.
**Figure 8****.** (A) miR-9 relative expression induced by TGF-β1 in omentum derived mesenchymal cells (MCs). (B) miR-9 relative expression in non epithelioid MCs derived from patients undergoing peritoneal dialysis compared to epithelioid MCs, and miR-9 relative expression in MCs isolated from effluents in dialysis fluid of patients undergoing continuous peritoneal dialysis compared to MCs from omentum. (C) Relative α-SMA, Col 1α1 and FN expression in MCs by TGF-β1-induced miR-9 overexpression. (D) α-SMA protein levels when miR-9 is upregulated.
**Figure 9****.** miR-9 expression in human dermal fibroblasts by RT-qPCR.
**Figure 10****.** TGFBRII mRNA (A) and protein (B) levels in human dermal fibroblasts transfected with miR-9 precursors.
**Figure 11****.** (A) mRNA levels of α-SMA, Col 1α1 and FN in human dermal fibroblasts when miR-9 expression is induced. (B) α-SMA and FN protein levels in human dermal fibroblasts upon induction of miR-9 expression.
**Figure 12****.** Western blot showing Smad 2 signaling upon miR-9 increased expression. Smad 2 phosphorylation attenuation in response to TGF-β1 stimulation in dermal fibroblasts.

### DETAILED DESCRIPTION OF THE INVENTION

### 1. Definitions

The term "administration", as used herein, relates to the delivery of a pharmaceutical drug to a patient. The skilled person knows suitable routes of administration according to a particular drug and include, without limitation, injection (e.g. intradermal, subcutaneous, intravenous, parenterally, intraperitoneally, intrathecally), intravesically (e.g. urinary bladder), intraprostatically, oral, inhalation, rectal and transdermal. In a particular embodiment, the agent for use according to the invention is administered oropharingeally.

The term "agent inducing expression" or "inducer agent", as used in this invention, refers to any molecule that is capable of causing an increase in the transcription of a gene. Usually, the gene the transcription of which is induced in response to said inducer agent is under the operative control of a transcription regulatory region which, in turn, has binding sites for a transcription activator the activity of which increases in the presence of said inducer agent. Preferably, the inducer agent is a compound that is easy to administer and easily distributed in the body, and innocuous at the doses used to activate the system. Moreover, it must be capable of penetrating into the desired tissue or organ, and have a suitable half-life (usually of several hours).

The term "diagnose" or "diagnosis", as used herein, relates to the evaluation of the probability according to which a subject suffers a specific pathology (in this case, a fibrotic disease, preferably an fibrotic disease selected from the group consisting of idiopathic pulmonary fibrosis, peritoneal fibrosis and skin fibrosis). As the skilled in the art will understand, such evaluation may not be correct for 100% of the subjects to be diagnosed, although it preferably is. The term, however, requires being able to identify a statistically significant part of the subjects.

The term "fibrotic disease", as used herein, relates to a disease involving fibrosis, which may be due to e. g. chronic inflammation or repair and reorganization of tissues. Fibrosis is the formation or development of excess fibrous connective tissue in an organ or tissue as a reparative or reactive process, as opposed to formation of fibrous tissue as a normal constituent of an organ or tissue, including the kidneys, heart, lungs, liver, skin and joints. Fibrotic diseases include, without limitation, pulmonary fibrosis (including idiopathic pulmonary fibrosis and cystic fibrosis), renal fibrosis, hepatic cirrhosis, endomyocardial fibrosis, atrial fibrosis, mediastinal fibrosis, myelofibrosis, retroperitoneal fibrosis, progressive massive fibrosis of the lungs, nephrogenic systemic fibrosis, Crohn's disease, keloid, Scleroderma/systemic sclerosis of the skin or the lungs, arthrofibrosis, Peyronie's disease, Dupuytren's contracture, some forms of adhesive capsulitis, peritoneal fibrosis, and skin fibrosis. In a particular embodiment, the fibrotic disease is selected from the group consisting of idiopathic pulmonary fibrosis, peritoneal fibrosis and skin fibrosis. In a preferred embodiment, the fibrotic disease is a pulmonary disease, in particular pulmonary fibrosis. The term "pulmonary disease" encompasses lung (pulmonary) fibrosis and pulmonary diseases with a fibrotic component selected from idiopathic pulmonary fibrosis, other interstitial pneumonias (IP) such as giant cell interstitial pneumonia, non-specific IP, cryptogenic organizing pneumonia, collagen vascular disease-associated IP, and drug-induced IP, also sarcodosis, cystic fibrosis, respiratory distress syndrome, granulomatosis, silicosis, asbestosis, systemic scleroderma involving the lung, as well as fibrosis and remodeling in asthma or chronic obstructive pulmonary disease (COPD). In a further embodiment, the fibrotic disease is generalized fibrosis, which is a scleroderma-like fibrosis that manifests itself in several target organs, including, without limitation, besides lung and/or skin, the spleen, heart, kidney and/or liver. In a particular embodiment, the fibrotic disease is idiopathic pulmonary fibrosis.

The term "idiopathic pulmonary fibrosis" or "IPF", as used herein, also known as "cryptogenic fibrosing alveolitis" (CFA) or "idiopathic fibrosing interstitial pneumonia", relates to a chronic, progressive and ultimately fatal disease characterized by a progressive decline in lung function. It is characterized by fibrosis of the supporting framework (interstitium) of the lungs. The term "idiopathic" is used only when the cause of the pulmonary fibrosis is unknown.

The term "increased expression level", as used herein in relation to the expression level of a miRNA, particularly miR-9, relates to a situation where the level of expression of miR-9 is increased at least 5%, at least 10%, at least 20%, at least 30%, at least 40%, at least 50%, at least 60%, at least 70%, at least 80%, at least 90% or at least 100% when compared to the corresponding reference value.

The term "level of expression" relates to the measurement of the amount of a nucleic acid, e.g. RNA or mRNA, or of a protein. In the context of the invention, the level of expression relates to the measurement of the amount of a nucleic acid, particularly RNA, more particularly miRNA, even more particularly miR-9.

The terms "miRNA" or "microRNA", used interchangeably herein, are endogenous RNAs, some of which are known to regulate the expression of protein-coding genes at the post-transcriptional level. As used herein, the term "miRNA" refers to any type of micro-interfering RNA, including but not limited to, endogenous microRNA and artificial microRNA. Typically, endogenous miRNAs are small RNAs encoded in the genome which are capable of modulating the productive utilization of mRNA. A mature miRNA is a single-stranded RNA molecule of about 21-23 nucleotides in length which is complementary to a target sequence, and hybridizes to the target RNA sequence to inhibit its translation. miRNAs themselves are encoded by genes that are transcribed from DNA but not translated into protein (non-coding RNA); instead they are processed from primary transcripts known as pri-miRNA to short stem-loop structures called pre-miRNA and finally to functional miRNA. Mature miRNA molecules are partially complementary to one or more messenger RNA (mRNA) molecules, and their main function is to down-regulate gene expression.

Pre-miRNAs are referred to as "mir", whereas mature miRNAs are referred to as "miR". This prefix is followed by a dash and a number, the latter often indicating order of naming. miRNAs with nearly identical sequences except for one or two nucleotides are annotated with an additional lower case letter. Pre-miRNAs that lead to 100% identical mature miRNAs but that are located at different places in the genome are indicated with an additional dash-number suffix. Species of origin is designated with a three-letter prefix. Nomenclature conventions used to describe miRNA sequences are described further in Ambros V et al., RNA, 2003, 9:277-279, the entire contents of which are incorporated herein by reference.

The term "miR-9", as used herein, refers to the human miRNA as defined in the miRBase database of the University of Manchester (http://www.mirbase.org/index.shtml) (release 20 as of June 2013) under accession number MIMAT0000441 (hsa-miR-9-5p, previously known as has-miR-9) and with sequence UCUUUGGUUAUCUAGCUGUAUGA (SEQ ID NO:1).

The term pre-miR-9, as used herein, refers to pre-miRNAs which are processed to provide miR-9. The sequences of these molecules are also available in the miRBase of the University of Manchester (release 20 as of June 2013) under accession numbers MI0000466 (hsa-mir-9-1, *Homo sapiens* miR-9-1 stem-loop), MI0000467 (hsa-mir-9-2, *Homo sapiens* miR-9-2 stem-loop) and MI0000468 (hsa-mir-9-3, *Homo sapiens* miR-9-3 stem-loop).

The term "peritoneal fibrosis", as used herein, relates to the substitution of the peritoneum by fibrous tissue secondary to peritonitis. It is characteristic in patients undergoing peritoneal dialysis programs having recurrent peritonitis and suffering from loss of efficacy of the peritoneal dialysate. Epithelial-mesenchymal transition (EMT) of peritoneal mesothelial cells plays a crucial role in peritoneal fibrosis. EMT is a process whereby fully differentiated epithelial cells undergo transition to a mesenchymal phenotype giving rise to fibroblasts and myofibroblasts.

The term "polynucleotide", as used herein, refers to a single-stranded or doublestranded DNA or RNA molecule comprising a number of nucleotide monomers covalently bonded. Preferably, the polynucleotide has 8 or more nucleotide monomers. In a preferred embodiment the polynucleotide is at least 8, at least 9, at least 10, at least 11, at least 12, at least 13, at least 14, at least 15, at least 16, at least 18, at least 20, at least 25, at least 30, at least 35, at least 40, at least 50, at least 60, at least 70, at least 80, at least 90, at least 100 or more nucleotides long.

The term "prevention", as used herein, relates to the capacity of a compound of the invention to prevent, minimize or hinder the onset or development of a disease or condition before its onset, wherein the disease according to the invention is a fibrotic disease.

The term "reference value" or "reference level", as used herein in the context of the method of the invention, relates to a laboratory value used as a reference for the expression level values/data obtained from samples of subjects to be diagnosed with a disease, in particular a fibrotic disease.

The term "sample" or "biological sample", as used herein, refers to biological material isolated from a subject. The biological sample contains any biological material suitable for detecting RNA, particularly miRNA, and is a material comprising genetic material from the subject. The biological sample can comprise cell and/or non-cell material of the subject. In the present invention, the sample comprises genetic material, e.g., DNA, genomic DNA (gDNA), complementary DNA (cDNA), RNA, heterogeneous nuclear RNA (hnRNA), mRNA, etc., from the subject under study. In a particular embodiment, the genetic material is RNA. In a preferred embodiment the RNA is miRNA. The sample can be isolated from any suitable tissue or biological fluid such as, for example blood, saliva, plasma, serum, urine, cerebrospinal liquid (CSF), feces, a buccal or buccal-pharyngeal swab, a surgical specimen, a specimen obtained from a biopsy, and a tissue sample embedded in paraffin. Methods for isolating samples are well known to those skilled in the art. In particular, methods for obtaining a sample from a biopsy include gross apportioning of a mass, or micro-dissection or other art-known cell-separation methods. In order to simplify conservation and handling of the samples, these can be formalin-fixed and paraffin-embedded or first frozen and then embedded in a cryosolidifiable medium, such as OCT-Compound, through immersion in a highly cryogenic medium that allows rapid freeze.

The term "skin fibrosis", as used herein, relates to fibrosis with excessive accumulation of extracellular matrix proteins in skin cells. It is induced by stimuli including persistent infections, autoimmune reactions or tissue injury that lead to an "excessive" wound healing. It is dependent on the activation of extracellular matrix synthesis in interstitial fibroblasts that evolve into α-smooth muscle actin-positive myofibroblasts.

The term "subject" or "individual" or "animal" or "patient" or "mammal," is meant any subject, particularly a mammalian subject, for whom diagnosis, prognosis, or therapy is desired. Mammalian subjects include humans, domestic animals, farm animals, and zoo, sports, or pet animals such as dogs, cats, guinea pigs, rabbits, rats, mice, horses, cattle, cows, and so on. In a preferred embodiment of the invention, the subject is a mammal. In a more preferred embodiment of the invention, the subject is a human.

The term "treatment", as used herein, refers to both therapeutic measures and prophylactic or preventative measures, wherein the object is to prevent or slow down (lessen) an undesired physiological change or disorder, such as a fibrotic disease. Beneficial or desired clinical results include, but are not limited to, alleviation of symptoms, diminishment of extent of disease, stabilized (i.e., not worsening) state of disease, delay or slowing of disease progression, amelioration or palliation of the disease state, and remission (whether partial or total), whether detectable or undetectable. "Treatment" can also mean prolonging survival as compared to expected survival if not receiving treatment. Those in need of treatment include those already with the condition or disorder as well as those prone to have the condition or disorder or those in which the condition or disorder is to be prevented.

The term "vector", as used herein, refers to a construct capable of delivering, and preferably additionally expressing, one or more polynucleotides of interest into a host cell. Examples of vectors include, but are not limited to, viral vectors, naked DNA or RNA expression vectors, plasmid, cosmid or phage vectors, DNA or RNA expression vectors associated with cationic condensing agents, DNA or RNA expression vectors encapsulated in liposomes, and certain eukaryotic cells, such as producer cells. This term also relates to targeting constructs which allow for random or site-directed integration of the targeting construct into genomic DNA. Such targeting constructs, preferably, comprise DNA of sufficient length for either homologous recombination or heterologous integration. In a particular embodiment, the polynucleotide encoding miR-9 for use according to the invention or the polynucleotide encoding pre-miR-9 for use according to the invention is provided within a lentiviral vector. The term "lentiviral vector" as used herein, relates to a nucleic acid sequence comprising the necessary sequences so that after transcribing and translating said sequences in a cell with expression of lentiviral genes a sequence of interest, corresponding to miR-9 or to pre-miR-9 according to the invention, is generated.

### 2. Agents inducing miR-9 expression for use in the treatments and/or prevention of idiopathic pulmonary fibrosis

The authors of the present invention have found that both oxidative stress and TGF-β1 induce miR-9 expression in pulmonary fibroblasts. Increasing miR-9 levels significantly reduced TGFBRII and TGF-β1-NOX4 induced expression in pulmonary fibroblasts. This effect correlated with a decrease in TGF-β1-mediated signaling. Up-regulation of miR-9 levels attenuated TGF-β-induced transformation of lung fibroblasts into myofibroblasts (see Example 1). These contractile fibroblasts are believed to be the main source of abnormal extracellular matrix production in organ fibrosis. Thus, the authors concluded that miR-9 down-regulates the profibrogenic activities of TGF-β1 *in vitro.* Assays performed in a murine model of lung fibrosis confirmed that the increase of miR-9 expression levels prevented bleomycin-induced lung fibrosis, as well as that miR-9 targets NOX4 and TGFBR2 (see Example 2). The inventors have also found that miR-9 plays a significant role in the TGF-β1 mediated transformation of mesenchymal cells into fibrogenic myofibroblasts, indicating a protective role for miR-9 in the initiation and progression of peritoneal fibrosis (see Example 4). Similarly, they have confirmed TGF-β1-induced miR-9 expression also in human dermal fibroblasts, as well as that miR-9 plays a significant role in the TGF-β1 mediated transformation of fibroblasts into fibrogenic myofibroblasts in skin fibrosis (see Example 5).

Thus, in a first aspect, the invention relates to an agent selected from the group consisting of miR-9 (accession number MIMAT0000441 according to miRBase database, SEQ ID NO:1), a miR-9 variant, an agent inducing the expression of miR-9 and an agent inducing the expression of a variant of miR-9, for use in the prevention and/or treatment of a fibrotic disease in a subject. Alternatively, the invention relates to the use of an agent selected from the group consisting of miR-9 (accession number MIMAT0000441 according to miRBase database, SEQ ID NO:1), a miR-9 variant, an agent inducing the expression of miR-9 and an agent inducing the expression of a variant of miR-9 for the manufacture of a medicament for the treatment and/or prevention of a fibrotic disease in a subject in need thereof. Alternatively, the invention relates to a method for the prevention and/or treatment of a fibrotic disease in a subject that comprises the administration to said subject of an agent selected from the group consisting of miR-9 (accession number MIMAT0000441 according to miRBase database, SEQ ID NO:1), a miR-9 variant, an agent inducing the expression of miR-9 and an agent inducing the expression of a variant of miR-9.

Thus, the agent for use according to the invention in the prevention and/or treatment of a fibrotic disease in a subject is selected from the group consisting of miR-9 (accession number MIMAT0000441 according to miRBase database, SEQ ID NO:1), a miR-9 variant, an agent inducing the expression of miR-9 and an agent inducing the expression of a variant of miR-9. Particularly, agents inducing expression of miR-9 for use according to the invention are selected from the group consisting of a pre-miR-9, a polynucleotide encoding miR-9 and a polynucleotide encoding pre-miR-9.

### 2.1. miR-9 and variants thereof

In an embodiment, the miR-9 microRNA for use according to the invention is the human miR-9, with accession number MIMAT0000441 (SEQ ID NO:1) according to the miRBase database of the University of Manchester. The miR-9 microRNA (homologous to miR-79), is a short non-coding RNA gene involved in gene regulation. The mature ∼21nt miRNAs are processed from hairpin precursor sequences by the Dicer enzyme. The dominant mature miRNA sequence is processed from the 5' arm of the mir-9 precursor, and from the 3' arm of the mir-79 precursor.

It will, of course, be understood that variants of said miR-9 can also be used in the context of the present invention. Said variants have a nucleic acid sequence which is at least 70 percent, at least 80 percent, at least 90 percent, at least 95 percent, at least 97, at least 98 percent or at least 99 percent identical to the sequence the miR-9, provided that these variant sequences also encode a functional miR-9.

Suitable methods for determining whether a given variant of the miR-9 can be used in the context of the present invention include, for instance, an *in vivo* method wherein the variant is administered to mice which have developed pulmonary fibrosis as a consequence of the treatment with bleomycin according to Example 2 of the present invention. Suitable variants for use according to the present invention include, without limitation, variants which substantially preserve the capacity of the miR-9 for preventing fibrogenic transformation of pulmonary fibroblasts, wherein said capacity is measured by determining the levels of different mRNAs which are increased in the presence of fibrosis, e.g. α-SMA, Col 1α1 and FN. Alternatively, miR-9 variants for use according to the present invention include those which are capable of attenuating TGF-β1-dependent transformation of fibroblasts into myofibroblasts as shown in Example 1 of the present invention. Suitable variants for use according to the present invention include, variants which show at least 99%, 98%, 97%, 96%, 95%, 94%, 93%, 92%, 91%, 90%, 80%, 70%, 60% or 50% of the capacity of the miR-9 for inhibiting fibrosis in the above mentioned *in vivo* or the capacity of attenuating TGF-β1-dependent transformation of fibroblasts into myofibroblasts in the above mentioned *in vitro* model.

Other variants which can be used in the method according to the present invention include polynucleotides which hybridize to the above polynucleotides, preferably, under stringent hybridization conditions provided that the variants retain the properties of a miR-9 as set forth above.

### 2.2. Agents inducing expression of miR-9 or of variants thereof

In an embodiment, the invention relates to an agent inducing the expression of miR-9 or to an agent inducing the expression of a variant of miR-9 for use in the prevention and/or treatment of a fibrotic disease in a subject. Variants of miR-9 according to the invention have been previously described.

Agents inducing the expression of miR-9 for use according to the invention comprise any molecule capable of increasing the expression of miR-9. Suitable methods for determining whether an agent is capable of increasing or up-regulating the levels of a miRNA (particularly of miR-9 or a variant thereof) include, without limitation, standard assays for determining mRNA expression levels such as qPCR, RT-PCR, RNA protection analysis, Northern blot, RNA dot blot, *in situ* hybridization, microarray technology, tag based methods such as serial analysis of gene expression (SAGE) including variants such as LongSAGE and SuperSAGE, microarrays, fluorescence in situ hybridization (FISH), including variants such as Flow-FISH, qFiSH and double fusion FISH (D-FISH), and the like.

In an embodiment of the invention, agents inducing expression of miR-9 for use according to the invention are selected from the group consisting of a pre-miR-9, a polynucleotide encoding miR-9 and a polynucleotide encoding pre-miR-9.

### 2.2.1. Agents inducing expression of miR-9: pre-miR-9

In an embodiment, the agent inducing expression of miR-9 for use according to the invention is a pre-miR-9, preferably a human pre-miR-9, or a variant thereof. In a particular embodiment, the pre-miR-9 for use according to the invention is selected from the group consisting of hsa-mir-9-1, hsa-mir-9-2 and hsa-mir-9-3.

hsa-mir-9-1 (*Homo sapiens* miR-9-1 stem-loop, accession number MI0000466 according to miRBase database, release 20 as of June 2013; HGNC:31641 according to HUGO Gene Nomenclature Commitee) is located at human chromosome 1q22. Homologs of hsa-mir-9-1 include, without limitation, *Rattus norvegicus* Mir3597-1, RGD:4888599 according to Rat Genome Database). The sequence of hsa-mir-9-1 is the following (SEQ ID NO:2): 
1 cgggguuggu uguuaucuuu gguuaucuag cuguaugagu gguguggagu cuucauaaag
61 cuagauaacc gaaaguaaaa auaacccca
 hsa-mir-9-2 (*Homo sapiens* miR-9-2 stem-loop, accession number MI0000467 according to miRBase database, release 20 as of June 2013; HGNC:31642 according to HUGO Gene Nomenclature Commitee) is located at human chromosome 5q14.3. The sequence of hsa-mir-9-2 is the following (SEQ ID NO:3): 
1 ggaagcgagu uguuaucuuu gguuaucuag cuguaugagu guauuggucu ucauaaagcu
61 agauaaccga aaguaaaaac uccuuca
 hsa-mir-9-3 (*Homo sapiens* miR-9-3 stem-loop, accession number MI0000468 according to miRBase database, release 20 as of June 2013; HGNC:31646 according to HUGO Gene Nomenclature Commitee) is located at human chromosome 15q26.1. Homologs of hsa-mir-9-3 include, without limitation, *Rattus norvegicus* Mir3597-3, RGD:4888559 according to Rat Genome Database). The sequence of hsa-mir-9-3 is the following (SEQ ID NO:4):

1 ggaggcccgu uucucucuuu gguuaucuag cuguaugagu gccacagagc cgucauaaag

61 cuagauaacc gaaaguagaa augauucuca

Synthetic exemplary pre-miR-9 include, without limitation, the Pre-miR™ miRNA precursors that are synthesized on demand by Ambion.

It will, of course, be understood that variants of said pre-miR-9 can also be used in the context of the present invention. Said variants have a nucleic acid sequence which is at least 70 percent, at least 80 percent, at least 90 percent, at least 95 percent, at least 97, at least 98 percent or at least 99 percent identical to the sequence the miR-9, provided that these variant sequences are processed to a functional miR-9 or a variant thereof. Suitable variants for use according to the present invention include, variants which encode miR-9 variants which show at least 99%, 98%, 97%, 96%, 95%, 94%, 93%, 92%, 91%, 90%, 80%, 70%, 60% or 50% of the capacity of the miR-9 for inhibiting fibrosis in the above mentioned *in vivo* or the capacity of attenuating TGF-β1-dependent transformation of fibroblasts into myofibroblasts in the above mentioned *in vitro* model.

Other variants which can be used in the method according to the present invention include polynucleotides which hybridize to the above polynucleotides, preferably, under stringent hybridization conditions provided that the variants retain the properties of a miR-9 as set forth above.

Other variants include polynucleotides which are identical to hsa-mir-9-1, hsa-mir-9-2 or hsa-mir-9-3 in the region which corresponds to the mature miR-9 but which differ in the region that forms in the 5' and 3' region which form the stem-loop and which is removed during processing to provide the mature miR-9.

### 2.2.2. Agents inducing expression of miR-9: polynucleotides encoding miR-9 or encoding pre-miR-9

In another embodiment, the agent inducing expression of miR-9 for use according to the invention is a polynucleotide encoding miR-9, preferably human miR-9 or a polynucleotide encoding pre-miR-9, preferably human miR-9. In a particular embodiment, the polynucleotide encoding miR-9 or the polynucleotide encoding pre-miR-9 is provided within a vector, preferably within a lentiviral vector.

In a particular embodiment, the polynucleotide encoding miR-9 encodes the miR-9 as defined under accession number MIMAT0000441 according to the miRBase database.

In a particular embodiment, the polynucleotide encoding pre-miR-9 encodes the human miR-9 according to accession numbers MI0000466 (hsa-mir-9-1, *Homo sapiens* miR-9-1 stem-loop), MI0000467 (hsa-mir-9-2, *Homo sapiens* miR-9-2 stem-loop) or MI0000468 (hsa-mir-9-3, *Homo sapiens* miR-9-3 stem-loop).

It will, of course, be understood that variants of said polynucleotide encoding pre-miR-are can also be used in the context of the present invention. Said variants have a nucleic acid sequence which is at least 70 percent, at least 80 percent, at least 90 percent, at least 95 percent, at least 97, at least 98 percent or at least 99 percent identical to the sequence of the polynucleotides encoding the miR-9 according to accession numbers MI0000466, MI0000467 or MI0000468, provided that these variant sequences also encode a pre-miR-9 which can be processed into a functional miR-9. Other variants which can be used in the method according to the present invention include polynucleotides which hybridize to the above polynucleotides, preferably, under stringent hybridization conditions provided that the variants retain the properties of a miR-9 as set forth above.

Preferably, the polynucleotide encoding miR-9 and/or the polynucleotide encoding pre-miR-9 further comprises from 200 to 400 base pairs upstream and/or downstream flanking the genomic sequence of said miR-9 or pre-miR-9, ensuring that the microRNA expressed from the polynucleotide for use according to the invention will be correctly processed in the cell into mature miR-9.

In a particular embodiment, the polynucleotide encoding miR-9 and/or the polynucleotide encoding pre-miR-9 for use according to the invention comprise(s) a transcriptional regulatory sequence. The term "transcriptional regulatory sequence", as used herein, refers to a nucleic acid sequence which is capable of governing the expression of another nucleic acid sequence operatively linked thereto, such as a nucleotide sequence encoding miR-9 or a nucleotide sequence encoding pre-miR-9. The transcription control sequence, preferably, is a DNA sequence. The transcriptional control sequence comprises a first promoter and, optionally, a binding site for a transcriptional repressor wherein said first promoter and said binding site for a transcriptional repressor are arranged so that the binding of the transcriptional repressor to said binding site inhibits the transcriptional activity of the promoter.

The term "promoter", as used herein, refers to a DNA sequence that determines the site of transcription initiation for an RNA polymerase. Promoter sequences comprise motifs which are recognized and bound by polypeptides, i.e. transcription factors. The said transcription factors shall upon binding recruit RNA polymerases II, preferably, RNA polymerase I, II or III, more preferably, RNA polymerase II or III, and most preferably, RNA polymerase II. Thereby will be initiated the expression of a nucleic acid operatively linked to the transcription control sequence. It is to be understood that dependent on the type of nucleic acid to be expressed, expression as meant herein may comprise transcription of DNA sequences into RNA polynucleotides (as suitable for, e.g., anti-sense approaches, RNAi approaches or ribozyme approaches) or may comprise transcription of DNA sequences into RNA polynucleotides followed by translation of the said RNA polynucleotides into polypeptides (as suitable for, e.g., gene expression and recombinant polypeptide production approaches). In order to govern expression of a nucleic acid sequence, the transcription control sequence may be located immediately adjacent to the nucleic acid to be expressed, i.e. physically linked to the said nucleic acid at its 5' end. Alternatively, it may be located in physical proximity. In the latter case, however, the sequence must be located so as to allow functional interaction with the nucleic acid to be expressed.

Suitable promoters for use as first promoters include any promoter known in the art. In a preferred embodiment, the first promoter is a promoter functional in mammalian cells. High-level constitutive promoters are preferred for use in the vectors according to the present invention. Examples of such promoters include, without limitation, the retroviral Rous sarcoma virus (RSN) LTR promoter (optionally with the RSV enhancer), the cytomegalovirus (CMV) promoter (optionally with the CMV enhancer), the SN40 promoter, the dihydrofolate reductase promoter, the beta-actin promoter, the beta-actine promoter linked to the enhancer derived from the cytomegalovirus immediate early (IE) promoter, the phosphoglycerol kinase (PGK) promoter, and the EFla promoter. Inducible promoters are regulated by exogenously supplied compounds, including, the zinc-inducible sheep metallothionine (MT) promoter, the dexamethasone (Dex)-inducible mouse mammary tumor virus (MMTV) promoter, the T7 polymerase promoter system; the ecdysone insect promoter, the tetracycline-repressible system, the tetracycline-inducible system, the RU486-inducible system and the rapamycin-inducible system. Other types of inducible promoters which may be useful in this invention are those which are regulated by a specific physiological state, e.g., temperature, acute phase, a particular differentiation state of the cell, or in replicating cells only.

In another embodiment, the native promoter for the genomic sequence encoding miR-9 or encoding pre-miR-9 will be used. The native promoter may be preferred when it is desired that expression of said genomic sequence encoding miR-9 or pre-miR-9 should mimic the native expression. The native promoter may be used when expression of said genomic sequence encoding miR-9 or pre-miR-9 must be regulated temporally or developmentally, or in a tissue-specific manner, or in response to specific transcriptional stimuli. In a further embodiment, other native expression control elements, such as enhancer elements, polyadenylation sites or Kozak consensus sequences may also be used to mimic the native expression. In another embodiment, the genomic sequence encoding miR-9 or pre-miR-9 or other desirable product to be expressed is operably linked to a tissue-specific promoter. For instance, if expression in skeletal muscle is desired, a promoter active in muscle should be used. These include the promoters from genes encoding skeletal α-actin, myosin light chain 2A, dystrophin, muscle creatine kinase, as well as synthetic muscle promoters with activities higher than naturally-occurring promoters. Examples of promoters that are tissue-specific are known for liver [albumin, Miyatake et al. J Virol, 71:5124-32 (1997); hepatitis B virus core promoter, Sandig et al, Gene Ther., 3:1002-9 (1996); and alpha-fetoprotein (AFP), Arbuthnot et al, Hum. Gene Ther, 7:1503-14 (1996)], bone [osteocalcin, Stein et al, Mol. Biol. Rep., 24:185-96 (1997); and bone sialoprotein, Chen et al, J Bone Miner. Res., 11:654-64 (1996)], lymphocytes [CD2, Hansal et al., J Immunol, 161:1063-8 (1998); immunoglobulin heavy chain; T cell receptor a chain], neuronal [neuron-specific enolase (NSE) promoter, Andersen et al. Cell. Mol. Neurobiol, 13:503-15 (1993); neurofilament light-chain gene, Piccioli et al., 1991, Proc. Natl. Acad. Sci. USA, 88:5611-5 (1991); the neuron-specific vgf gene, Piccioli et al, Neuron 15:373-84 (1995)], lung [surfactant protein A, Bruno et al, J Biol Chem, 270, 6531-6536 (1996); surfactant protein B, Margana and Boggaram, J Biol Chem, 272, 3083-3090 (1997); surfactant protein C, Glasser et al, Am J Physiol, 261, L349-356 (1991)], skin [keratin K14, Staggers et al, Gene, 153, 297-298 (1995)], kidney [Na+/glucose cotransporter gene, Kanai et al, J Clin Invest, 93(1), 397-404 (1994); Ksp-cadherin gene, Igarashi et al, Am J Physiol, 277(4 Pt 2), F599-610 (1999); Na+/K+/Cl cotransporter gene, Igarashi et al, J Biol Chem, 271(16):9666-74 (1996); Podocin, Buchholz et al, Nucleic Acids Res, 24(21):4256-62 (1996); γ-glutamyl transpeptidase, Indra et al, Nucleic Acids Res, 27(22), 4324-7 (1999)]; among others.

Preferably, a promoter referred to herein can be derived from the cytomegalovirus (CMV) minimal promoter and, more preferably, from human CMV (hCMV) such as the hCMV immediate early promoter derived minimal promoter as described in, e.g., Gossen and Bujard (Proc. Natl. Acad. Sci. USA, 1992, 89: 5547-5551). Modified promoters also may be used, including insertion and deletion mutation of native promoters and combinations or permutations thereof.

Alternatively, the polynucleotide encoding miR-9 or the polynucleotide encoding pre-miR-9 for use according to the invention may further comprise a polynucleotide of interest under operative control of the transcriptional regulatory region. The skilled person will appreciate that the polynucleotides of interest for use in the polynucleotides according to the present invention will depend on the intended use of the vector. Particularly preferred polynucleotides include reporter genes, when the polynucleotides of the invention are used as research tools or for imaging purposes, polynuclotides encoding polypeptides when the polynucleotides are used for gene therapy in diseases characterised by a lack of function of said polypeptide and polynucleotides which are capable of silencing the expression of target genes, adequate in those cases wherein the polynucleotides according to the invention are used for the treatment of diseases associated with an excessive expression of a given gene, either endogenous to the organism being treated or heterologous.

In a particular embodiment, the polynucleotide encoding miR-9 or the polynucleotide encoding pre-miR-9 for use according to the invention, as previously described, is provided within a vector.

Preferably, the vector comprising the polynucleotide encoding miR-9 or the polynucleotide encoding pre-miR-9 for use according to the present invention further comprises selectable markers for propagation and/or selection in a host.

A vector may be characterized by one or a small number of restriction endonuclease sites at which such DNA sequences may be cut in a determinable fashion without the loss of an essential biological function of the vector, and into which a DNA fragment may be spliced in order to bring about its replication and cloning. A vector may further contain a marker suitable for use in the identification of cells transformed with the vector.

The vector for use according to the present invention usually comprises regions adequate for inserting a polynucleotide of interest (e.g. the polynucleotide encoding miR-9 or pre-miR-9), so that said polynucleotide is under operative control of the transcriptional regulatory region. In a preferred embodiment, the polynucleotide encoding miR-9 for use according to the invention comprises a polylinker downstream of the transcriptional regulatory region. The terms "polylinker" and "multiple cloning site" are used herein interchangeably to refer to refer to a region of DNA that contains one or more commonly used restriction sites allowing the insertion of a DNA fragment (e.g., gene of interest) into the expression construct.

One type of vector is a "plasmid", which refers to a circular double stranded DNA loop into which additional DNA segments can be ligated. Another type of vector is a viral vector, wherein additional DNA segments can be ligated into the viral genome. Certain vectors are capable of autonomous replication in a host cell into which they are introduced (e. g., bacterial vectors having a bacterial origin of replication and episomal mammalian vectors). Other vectors (e. g., non-episomal mammalian vectors) are integrated into the genome of a host cell upon introduction into the host cell, and thereby are replicated along with the host genome. Moreover, certain vectors are capable of directing the expression of genes to which they are operatively linked. Such vectors are referred to herein as "expression vectors". In general, expression vectors of utility in recombinant DNA techniques are often in the form of plasmids. In the present specification, "plasmid" and "vector" can be used interchangeably as the plasmid is the most commonly used form of vector. However, the invention is intended to include such other forms of expression vectors, such as viral vectors (e. g., replication defective retroviruses, adenoviruses and adeno-associated viruses), which serve equivalent functions.

In a preferred embodiment of the vector of the present invention, said vector is an expression vector. More preferably, in the vector of the invention, the transcription control sequence is operatively linked to a nucleic acid sequence to be expressed. Such operative linkage, preferably, allows expression of the said nucleic acid sequence in eukaryotic cells or isolated fractions thereof. In principle, regulatory elements ensuring expression in eukaryotic cells, preferably mammalian cells, are well known in the art. They, preferably, comprise regulatory sequences ensuring initiation of transcription as comprised by the transcription control sequence of the present invention as well as poly-A signals ensuring termination of transcription and stabilization of the transcript. Additional regulatory elements may be included into the vector such as transcriptional as well as translational enhancers. In this context, suitable expression vectors are known in the art such as vectors derived from retroviruses including lentiviruses, adenovirus, cytomegalovirus, adeno-associated viruses, measles virus, vaccinia virus, herpes viruses, or bovine papilloma virus, may be used for delivery of the vector of the invention into targeted cell population. Methods which are well known to those skilled in the art can be used to construct recombinant viral vectors.

Among the different vectors that may be used as all-in-one vectors in the context of the invention, lentiviral vectors are preferred because they are one of the most promising vectors for gene transfer in primary human cells, they are highly efficient and do not express any viral gene that could alter normal cellular physiology.

As used herein, the term "lentivirus" refers to a group (or scientific genus) of retroviruses that in nature give rise to slowly developing disease due to their ability to incorporate into a host genome. Modified lentiviral genomes are useful as viral vectors for the delivery of a nucleic acid sequence to a cell. An advantage of lentiviruses for infection of cells is the ability for sustained transgene expression. These viruses include in particular Human Immunodeficiency Virus type 1 (HIV-1), Human Immunodeficiency Virus type 2 (HIV-2), Simian Immunodeficiency Virus (SIV), Feline Immunodeficiency Virus (FIV), Equine Infectious Anaemia Virus (EIAV), Bovine Immunodeficiency Virus (BIV), Visna Virus of sheep (VISNA) and Caprine Arthritis-Encephalitis Virus (CAEV). Lentiviral vectors are well known in the art (see, for example, U.S. Pat. Nos. 6,013,516 and 5,994,136).

Lentiviral vectors are well known in the art (see, for example, U.S. Pat. Nos. 6,013,516 and 5,994,136). Recombinant lentiviral vectors are capable of infecting non-dividing cells and can be used for both *in vivo* and *ex vivo* gene transfer and expression of nucleic acid sequences. For example, recombinant lentiviruses capable of infecting a non-dividing cell wherein a suitable host cell is transfected with two or more vectors carrying the packaging functions, namely *gag, pol* and *env,* as well as *rev* and *tat* is described in U.S. Pat. No. 5,994,136. One may target the recombinant virus by linkage of the envelope protein with an antibody or a particular ligand for targeting to a receptor of a particular cell-type. By inserting a sequence (including a regulatory region) of interest into the viral vector, particularly a polynucleotide encoding miR-9 or a polynucleotide encoding pre-miR-9, along with another gene which encodes the ligand for a receptor on a specific target cell, for example, the vector is now target-specific. The recombinant lentiviruses according to the invention may be genetically modified in such a way that certain genes constituting the native infectious virus are eliminated and replaced with a nucleic acid sequence of interest to be introduced into the target cells.

The lentiviral vector can integrate into the genome of the host cell. The genetic material thus transferred is then transcribed and possibly translated into proteins inside the host cell. When the lentiviral vector is a non-integrative lentiviral vector, the vector is present in episomal forms. The lentiviral vector according to the present invention, in addition to the polynucleotide encoding miR-9, the polynycleotide encoding pre-miR-9, and/or the transcriptional regulatory sequence as described above, may further comprise additional elements which help to improve expression of the nucleotide sequence, particularly miRNA sequence, encoded within the vector. Additional elements include:
- Regions required for the integration of the vector into the genome of the target cell such as the Long-terminal repeats (LTRs) which flank the all-in-one vector defined herein. Thus, a lentiviral vector for use according to the invention preferably comprises a 5' LTR and a 3' LTR. "5' LTR" refers to a 5' retroviral or lentiviral long terminal repeat, which may or may not be modified from its corresponding native 5' LTR by deleting and/or mutating endogenous sequences and/or adding heterologous sequences. The 5' LTR may be natural or synthetic. "3' LTR" refers to a 3' retroviral or lentiviral long terminal repeat, which may or may not be modified from its corresponding native (i.e., that existing in the wild-type retrovirus) 3' LTR by deleting and/or mutating endogenous sequences and/or adding heterologous sequences. The 3' LTR may be natural or synthetic.
- An encapsidation sequence such as the lentiviral Psi (ψ) sequence,
- Sequences enhancing the RNA nuclear export, advantageous during the production, such as the sequence comprising the HIV-1 REV response element (RRE) sequence. Another sequence, usable in the context of the present invention, which enhances the RNA nuclear export, is the CTE sequence (Oh et al, 2007, Retrovirology. 2007 Jun 5;4:38.). These sequences are also useful for determining the copy number of the integrated lentiviral vectors.
- Sequences enhancing the nuclear import of the retrotranscribed viral DNA, such as the the lentiviral cPPT CTS (flap) sequence from, without limitation, HIV-1, HIV-2, SIV, FIV, EIAV, BIV, VISNA and CAEV.
- Posttranscriptional regulation elements may be selected from Woodchuck hepatitis virus responsive element (WPRE), APP UTR5' region and TAU UTR3'.
- One or more insulator sequences selected from the group consisting of, for example, MAR, SAR, S/MAR, scs and scs' sequences.
- Additional transcriptional regulatory elements: These are regions which help to improve expression of the genes encoded within the vector. In particular, the vector may incorporate the wood-chuck hepatitis virus post-transcriptional regulatory element (WPRE) at the 3' untranslated region (Paterna et al., 2000, Gene Ther. 7: 1304-1311).

In another embodiment, the lentiviral vector is another form of self-inactivating (SIN) vector as a result of a deletion in the 3' long terminal repeat region (LTR). Preferably, the vector contains a deletion within the viral promoter. The LTR of lentiviruses such as the HIV LTR contains a viral promoter. Although this promoter is relatively inefficient, when transactivated by e.g. tat, the promoter is efficient because tat-mediated transactivation increases the rate of transcription about 100 fold. However, the presence of the viral promoter can interfere with heterologous promoters operably linked to a transgene. To minimize such interference and better regulate the expression of transgenes, the lentiviral promoter may be deleted.

In a particular embodiment, the lentiviral vector comprises, in the 5' to 3' orientation:
- a WPRE element, that enhances stability and translation of the CMV-driven transcripts,
- a SV40 polyadenylation signal, that enables efficient termination of transcription and processing of recombinant transcripts,
- a hybrid RSV-5'LTR promoter, that provides a high level of expression of the full-length viral transcript in producer cells, such as 293 cells,
- some genetic elements (cPPT, GAG, LTRs), which are necessary for packaging, transducing, and stably integrating the viral expression construct into genomic DNA,
- a SV40 origin, for stable propagation of the plasmid in mammalian cells,
- a pUC origin, for high copy replication and maintenance of the plasmid in *E. coli* cells,
- an ampicillin resistance gene, for selection in *E. coli* cells,
- a fluorescent marker such as GFP, to monitor cells positive for transfection and transduction, and
- a resistance gene, such as a a puromycin resistant gene for puromycin selection marker, to enrich transduced cells and establish stable cell lines.

miRNA expression vectors are commercially allowable and include, without limitation, miRNA expression vectors pmR-ZsGreenl (catalog No. 632541) and pmR-mCherry Vector (catalog No. 632542) from Clontech, pMIR-REPORT™ miRNA Expression Reporter Vector System (catalog No. AM5795) from Life Technologies, HIV-based or FIV-based lentivectors from System Biosciences, and miRNA mammalian expression vectors (catalog Nos. MIR-EXP-GP-C and MIR-EXP-C) from Cell Biolabs. Lentiviral vectors carrying miRNA libraries such as the pLemiR plasmid have been described (Open Biosystems) wherein the sequence encoding the miRNA is under the control of a CMV promoter. Since these plasmids encode for the precursor form of the miRNA (the pri-miRNA), activation by the cellular machinery is required before the miRNA can exert its biological effects.

Lentiviral expression vectors for human pre-miR-9 expression are also commercially available and include, without limitation, the human pre-microRNA Expression Construct Lenti-miR-9-1 (Catalog No. PMIRH9-1PA-1, for human precursor under accession number MI0000466) and human pre-microRNA Expression Construct Lenti-miR-9-2 (Catalog No. PMIRH9-2PA-1, for human precursor accession number MI0000467) from System Biosciences.

### 2.3. Fibrotic diseases to be presented and/or treated by the agents for use according to the invention

Agents according to the invention as described above are used in the prevention and/or treatment of a fibrotic disease in a subject. In a particular embodiment, the fibrotic disease is selected from the group consisting of idiopathic pulmonary fibrosis, peritoneal fibrosis and skin fibrosis. In a more particular embodiment, the fibrotic disease is idiopathic pulmonary fibrosis. In a particular embodiment, the subject is a human.

Prevention and/or treatment of peritoneal fibrosis in a subject in need thereof may be followed by methods known by the skilled person including, without limitation, determination of one or more parameters used to monitor the functional status of the peritoneal membrane, including dialysate cancer antigen CA125 concentration and transport parameters.

Prevention and/or treatment of skin fibrosis in a subject in need thereof may be followed by methods known by the skilled person including, without limitation, profilometric analysis and elasticity tests. Profilometric analysis comprises skin roughness criteria including RA (average roughness), RZ (average depth of furrows), RS (residual length), RN (number of wrinkles) and AR (space between wrinkles). The elasticity test is performed using a LPG® tester, connected to a flexible coupling and linked to an aspiration system.

Prevention and/or treatment of an idiopathic pulmonary fibrosis in a subject in need thereof may be followed by determining one or more pulmonary function parameters. Pulmonary function parameters are known by the skilled person and include, without limitation, vital capacity (VC), residual volume (RV), forced expiratory volume (FEV), forced vital capacity (FVC), forced vital capacity percent (FVC%), forced inspiratory flow (FIF), peak expiratory flow rate (PEFR), inspiratory reserve volume (IRV), functional residual capacity (FRC), inspiratory capacity (IC), total lung capacity (TLC), expiratory reserve volume (ERV), tidal volume (TV), maximum voluntary ventilation (MVV), forced vital capacity (FVC), as follows:
- vital capacity (VC) is the total volume of air that can be moved in and out of the lungs. VC is equal to the combined inspiratory reserve volume, tidal volume, and expiratory reserve volume,
- residual volume (RV) is the volume of air remaining in the lungs after a maximal exhalation,
- forced expiratory volume (FEV) is the expiratory volume of air from a maximally forced expiratory effort, usually measured over a set period of time, e.g., 1 second, FEV1; 6 seconds, FEV6; etc.
- forced inspiratory flow (FIF) is the inspiratory volume of air from a maximally forced inspiratory effort, usually measured over a set period of time, e.g., 1 second, FIF 1 ; 6 seconds, FIF6; etc.
- peak expiratory flow rate (PEFR) is the highest forced expiration flow rate,
- inspiratory reserve volume (IRV) is the maximal volume that can be inhaled after a normal inspiration, measured from the end-inspiratory level,
- functional residual capacity (FRC) is the sum of the expiratory reserve volume and the residual volume. Typically, FRC represents the volume of air in the lungs at the end of a normal expiration,
- inspiratory capacity (IC) is the sum of the inspiratory reserve volume and the tidal volume,
- total lung capacity (TLC) is the sum of the vital capacity arid residual volume that represents the total volume of air that can be contained in the lung,
- expiratory reserve volume (ERV) is the maximal volume of air that can be exhaled after a normal expiration, measured from the end-expiratory position.
- tidal volume (TV) is the volume of air inhaled or exhaled during one respiratory cycle, typically measured at rest,
- maximum voluntary ventilation (MVV) is the volume of air expired in a specified time period during repetitive maximal effort,
- forced vital capacity (FVC) is the vital capacity from a maximally forced expiratory effort; FVC% is a subject's measured FVC expressed as the percentage of the predicted FVC for the subject. As used herein, all FVC% predicted values are absolute values and not relative values, and
- FEV1/FVG ratio means the ratio between forced expiratory volume in one second and forced vital capacity.

Many of these pulmonary function parameters are readily obtainable through the use of a spirometer as is well-known in the art. Residual volume can be obtained through indirect methods such as radiographic planimetry, body plethysmography, closed circuit dilution (including the helium dilution technique), and nitrogen washout.

### 2.4. Administration routes of the agents for use according to the invention

The administration of the agent for use of the invention can be performed by different routes, for instance, intravenous, intraperitoneal, subcutaneous, intramuscular, topical, intradermal, intranasal or intrabronchial, and it can be administered locally or systemically or directly to the target site. A review of the different administration routes of active substances, of the excipients to be used and the manufacturing procedures can be found in Tratado de Farmacia Galénica, C. Faulli i Trillo, Luzán 5, S.A. de Ediciones, 1993 and in Remington's Pharmaceutical Sciences (A.R. Gennaro, Ed.), 20th edition, Williams & Wilkins PA, USA (2000). The agent for use of the invention can occur at any pharmaceutical form of administration considered appropriate for the selected administration route, for example, by systemic, oral, parenteral or topical administration, for which it will include the pharmaceutically acceptable excipients necessary for formulation of the desired method of administration.

The dosage regimen will be established by the physician and the clinical factors. As it is well known in medicine, the dosages depend on many factors, including the physical characteristics of the patient (age, size, sex), the administration route used, the severity of the disease, the particular compound used and the pharmacokinetic properties of the subject.

The effective quantity of the compounds of the invention can vary within a wide range and, in general, will vary depending on the particular circumstances of application, duration of the exposure and other considerations.

Solid dosage forms for oral administration may include conventional capsules, sustained release capsules, conventional tablets, sustained-release tablets, chewable tablets, sublingual tablets, effervescent tablets, pills, suspensions, powders, granules and gels. At these solid dosage forms, the active compounds can be mixed with at least one inert excipient such as sucrose, lactose or starch. Such dosage forms can also comprise, as in normal practice, additional substances other than inert diluents, e.g. lubricating agents such as magnesium stearate. In the case of capsules, tablets, effervescent tablets and pills, the dosage forms may also comprise buffering agents. Tablets and pills can be prepared with enteric coatings.

Liquid dosage forms for oral administration may include emulsions, solutions, suspensions, syrups and elixirs pharmaceutically acceptable containing inert diluents commonly used in the technique, such as water. Those compositions may also comprise adjuvants such as wetting agents, emulsifying and suspending agents, and sweetening agents, flavoring and perfuming agents.

Injectable preparations, for example, aqueous or oleaginous suspensions, sterile injectable may be formulated according with the technique known using suitable dispersing agents, wetting agents and/or suspending agents. Among the acceptable vehicles and solvents that can be used are water, Ringer's solution and isotonic sodium chloride solution. Sterile oils are also conventionally used as solvents or suspending media.

For topical administration, compounds of the invention can be formulated as creams, gels, lotions, liquids, pomades, spray solutions, dispersions, solid bars, emulsions, microemulsions and similars which may be formulated according to conventional methods that use suitable excipients, such as, for example, emulsifiers, surfactants, thickening agents, coloring agents and combinations of two or more thereof.

Additionally, the compounds of the invention may be administered in the form of transdermal patches or iontophoresis devices. In one embodiment, the compounds of the invention are administered as a transdermal patch, for example, in the form of sustained-release transdermal patch. Suitable transdermal patches are described in more detail in, for example, US5262165, US5948433, US6010715 and US6071531.

The compositions comprising the compounds of the invention can additionally include conventional excipients, i.e. pharmaceutically acceptable carriers suitable for parenteral application which do not react damaging with the active compounds. Suitable pharmaceutically acceptable vehicles include, for example, water, salt solutions, alcohol, vegetable oils, polyethylene glycols, gelatin, lactose, amylose, magnesium stearate, talc, surfactants, silicic acid, viscous paraffin, perfume oil, monoglycerides and diglycerides of fatty acids, fatty acid esters petroetrals, hydroxymethyl cellulose, polyvinylpyrrolidone and similars.

Several drug delivery systems are known and can be used to administer the compounds or compositions of the invention, including, for example, encapsulation in liposomes, microbubbles, emulsions, microparticles, microcapsules and similars. The required dosage can be administered as a single unit or in a sustained release form.

Sustainable-release forms and appropriate materials and methods for their preparation are described in, for example, "Modified-Release Drug Delivery Technology", Rathbone, M. J. Hadgraft, J. and Roberts, M. S. (eds.), Marcel Dekker, Inc., New York (2002), "Handbook of Pharmaceutical Controlled Release Technology", Wise, D. L. (ed.), Marcel Dekker, Inc. New York, (2000). In one embodiment of the invention, the orally administrable form of a compound according to the invention is in a sustained release form further comprises at least one coating or matrix. The coating or sustained release matrix include, without limitation, natural polymers, semisynthetic or synthetic water-insoluble, modified, waxes, fats, fatty alcohols, fatty acids, natural semisynthetic or synthetic plasticizers, or a combination of two or more of them.

Enteric coatings may be applied using conventional processes known to experts in the art, as described in, for example, Johnson, J. L., "Pharmaceutical tablet coating", Coatings Technology Handbook (Second Edition), Satas, D. and Tracton, A. A. (eds), Marcel Dekker, Inc. New York, (2001), Carstensen, T., "Coating Tablets in Advanced Pharmaceutical Solids", Swarbrick, J. (ed.), Marcel Dekker, Inc. New York (2001), 455-468.

Even though individual needs vary, determination of optimal ranges for effective amounts of the compound of the invention belongs to the common experience of those experts in the art. In general, the dosage needed to provide an effective amount of such compound, which can be adjusted by one expert in the art will vary depending on age, health, fitness, sex, diet, weight, degree of alteration of the receptor, frequency of treatment and the nature and extent of impairment or illness, medical condition of the patient, route of administration, pharmacological considerations such as activity, efficacy, pharmacokinetic and toxicology profile of the particular compound used, if using a system drug delivery, and if the compound is administered as part of a combination of drugs.

The amount of the compound according to the invention that is effective in the treatment of a particular disorder or condition will depend on the nature of the disorder or condition, in particular a fibrotic disease, and can be determined by conventional clinical techniques, including reference to Goodman and Gilman, supra; The Physician's Desk Reference, Medical Economics Company, Inc., Oradell, NJ, 1995, and Drug Facts and Comparisons, Inc., St. Louis, MO, 1993. The precise dose to use in the formulation will also depend on the route of administration, and severity of the disease or disorder, and should be decided in the physician's opinion and the patient's circumstances.

In the particular case that the agent for use according to the invention is miR-9 or pre-miR-9, it is formulated in a composition intended for use in gene therapy. In the particular case that the agent for use according to the invention is selected from the group consisting of a polynucleotide encoding miR-9 or a polynucleotide encoding pre-miR-9, by way of illustration and not limitation, it may be formulated in a pharmaceutical composition containing a viral or non-viral vector. By way of illustration and not limitation, said vectors, may be viral, for example, based on retrovirus, adenovirus, etc., or nonviral such as ADN-liposome, ADN-polymer, ADN-polymer-liposome complexes, etc. [see "Nonviral Vectors for Gene Therapy", edited by Huang, Hung and Wagner, Academic Press (1999)]. Said vectors, which contain a polynucleotide according to the invention, may be administered directly to humans or animals by conventional methods. Alternatively, said vectors may be used to transform, or transfect or infect cells, for example, mammal cells, including human, *ex vivo,* which subsequently will be implanted into a human body or an animal to obtain the desired therapeutic effect. For administration to a human body or an animal, said cells will be formulated in a suitable medium that will have no adverse influence on cell viability.

In a particular embodiment, the agent for use according to the invention in the prevention and/or treatment of a fibrotic disease in a subject is administered oropharingeally to said subject.

### 3. Method for diagnosing a fibrotic disease

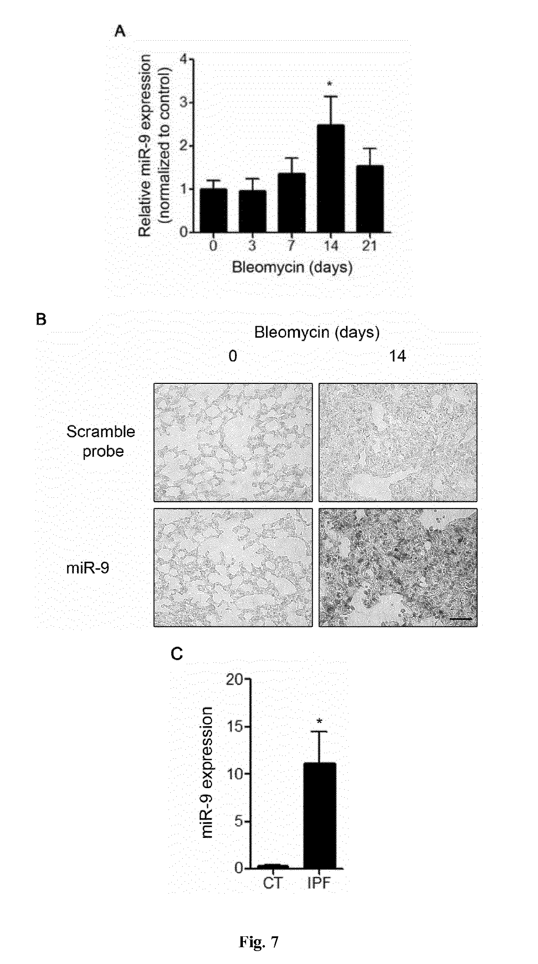

The inventors have found that miR-9 expression levels in lung samples were increased in patients suffering from idiopathic pulmonary fibrosis (IPF, n=7) when compared to healthy subjets (n=3) (see Example 3 and Figure 7).

Thus, in a further aspect, the invention relates to a method for diagnosing a fibrotic disease in a subject, hereinafter referred to as "diagnostic method of the invention", that comprises:
(i) determining the level of expression of miR-9 in a sample from said subject, and
(ii) comparing the level of expression in (i) to a reference value,
wherein if the expression level of miR-9 is increased with respect to the reference value, then the subject is diagnosed with a fibrotic disease.

Thus, in a first step, the diagnostic method of the invention comprises determining the level of expression of miR-9 in a sample from a subject whose diagnosis is to be determined.

Before analyzing the sample, it will often be desirable to perform one or more preparation operations upon said sample aimed at separating the molecule to be determined (RNA, in particular miRNA) from other molecules found in the sample. Typically, these sample preparation operations include manipulations such as concentration, suspension, extraction of intracellular material, e.g., nucleic acids from tissue/whole cell samples and the like, amplification of nucleic acids, fragmentation, transcription, labeling and/or extension reactions. Nucleic acids, especially RNA can be isolated using any techniques known in the art. There are two main methods for isolating RNA: (i) phenol-based extraction and (ii) silica matrix or glass fiber filter (GFF)-based binding. Phenol-based reagents contain a combination of denaturants and RNase inhibitors for cell and tissue disruption and subsequent separation of RNA from contaminants. Phenol-based isolation procedures can recover RNA species in the 10-200-nucleotide range e.g., miRNAs, 5S rRNA, 5.8S rRNA, and U1 snRNA. If a sample of "total" RNA was purified by the popular silica matrix column or GFF procedure, it may be depleted in small RNAs. Extraction procedures such as those using Trizol or TriReagent, however will purify all RNAs, large and small, and are the recommended methods for isolating total RNA from biological samples that will contain miRNAs/siRNAs. These methods are typically well known by a person skilled in the art. There are also commercial kits available for miRNA purification including, without limitation, miRNeasy Mini kit from Qiagen, miRNA isolation kits from Life Technologies, mirPremier microRNA isolation kit from Sigma-Aldrich and High Pure miRNA isolation kit from Roche.

The level of expression of miR-9 is determined in a sample from a subject, wherein said sample is any sample comprising nucleic acids comprising, without limitation, a cell sample (i.e. a lung cell sample), a tissue sample (i.e. a lung tissue sample), a bronchoalveolar lavage sample, a serum sample and a urine sample. The sample can be obtained by any conventional method depending on the sample to be analyzed. Methods for obtaining said samples are routinary and known by the skilled person. In a particular embodiment, the sample is a lung tissue sample.

The expression level can be determined using RNA obtained from a formalin-fixed, paraffin-embedded tissue sample. RNA may be isolated from an archival pathological sample or biopsy sample which is first deparaffinized. An exemplary deparaffinization method involves washing the paraffinized sample with an organic solvent, such as xylene. Deparaffinized samples can be rehydrated with an aqueous solution of a lower alcohol. Suitable lower alcohols, for example, include methanol, ethanol, propanols and butanols. Deparaffinized samples may be rehydrated with successive washes with lower alcoholic solutions of decreasing concentration, for example. Alternatively, the sample is simultaneously deparaffinized and rehydrated. The sample is then lysed and RNA is extracted from the sample. Samples can be also obtained from fresh tumor tissue such as a resected tumor.

Determination of the levels of RNA, in particular the levels of miRNA, can be carried out by any method known in the art such as qPCR, northern blot, RNA dot blot, TaqMan, tag based methods such as serial analysis of gene expression (SAGE) including variants such as LongSAGE and SuperSAGE, microarrays. Determination of the mRNA levels can also be carried out by fluorescence in situ hybridization (FISH). The detection can be carried out in individual samples or in tissue microarrays. In a particular embodiment, the expression levels of miR-9 in a sample from a subject to be diagnosed with a fibrotic disease are determined by real-time PCR. Methods to determine the expression levels of miR-9 in a sample have been previously described in the context of the agent for use according to the present invention. In a particular embodiment, the expression levels of miR-9 in a sample from a subject to be diagnosed with a fibrotic disease are determined by real-time PCR.

In order to normalize the values of expression of miRNA among the different samples, it is possible to compare the expression levels of the miRNA of interest in the test samples with the expression of a control RNA. Thus, in the context of the invention, the term "normalized" expression level refers to the expression level of a miRNA, particularly miR-9, relative to the expression level of a single reference gene or control RNA, or a particular set of reference or control RNAs. A "control RNA" as used herein, relates to RNA whose expression level does not change or changes only in limited amounts in samples under analysis with respect to control samples. Preferably, the control RNA is mRNA derived from housekeeping genes and which code for proteins which are constitutively expressed and carry out essential cellular functions. Preferred housekeeping genes for use in the present invention include β-2-microglobulin, ubiquitin, ribosomal protein 18S, cyclophilin, IPO8, HPRT, GAPDH, PSMB4, tubulin, transferrin receptor, tyrosine 3-monooxygenase/tryptophan 5-monooxygenase activation protein (YWHAZ) and β-actin.

In one embodiment, relative miRNA expression quantification is calculated according to the comparative threshold cycle (Ct) method using GAPDH, IPO8, HPRT, β-actin or PSMB4 as an endogenous control and commercial RNA controls as calibrators. Final results are determined according to the formula 2-(ΔCt sample-ΔCt calibrator), where ΔCT values of the calibrator and sample are determined by subtracting the Ct value of the target gene from the value of the control gene.

In a second step of the diagnostic method of the invention, the level of expression of miR-9 in a sample from a subject to be diagnosed is compared to a reference value, wherein if the expression level of miR-9 is increased with respect to the reference value, then the subject is diagnosed with a fibrotic disease.

The reference value or reference level according to the method of the invention can be an absolute value; a relative value; a value that has an upper and/or lower limit; a range of values; an average value; a median value, a mean value, or a value as compared to a particular control or baseline value. A reference value can be based on an individual sample value, such as for example, a value obtained from a sample from the subject being tested, but at an earlier point in time. The reference value can be based on a large number of samples, such as from a population of subjects of the chronological age matched group, or based on a pool of samples including or excluding the sample to be tested. Various considerations are taken into account when determining the reference value of the marker. Among such considerations are the age, weight, sex, general physical condition of the patient and the like. For example, equal amounts of a group of at least 2, at least 10, at least 100 to preferably more than 1000 subjects, preferably classified according to the foregoing considerations, for example according to various age categories, are taken as the reference group.

In a particular embodiment of the diagnostic method of the invention, the reference value is the expression level of the miR-9 determined in a sample from a healthy subject or in a sample from a subject who has not been diagnosed with a fibrotic disease, preferably in a sample from a healthy subject. In another embodiment, the reference value is the expression level of the miR-9 is an average value determined in a pool of healthy subjects or in a pool of healthy subjects who have not been diagnosed with a fibrotic disease, preferably in a pool of healthy subjects. Said reference value sample is typically obtained by combining equal amounts of samples from a subject population.

Once the reference value of miR-9 expression is established, the expression levels of miR-9 expressed in a sample from a subject to be diagnosed can be compared with this reference value, and thus be assigned a level of "increased" or "decreased". For example, an increase in expression levels above the reference value of at least 1.1-fold, 1.5-fold, 5-fold, 10-fold, 20-fold, 30-fold, 40-fold, 50-fold, 60-fold, 70-fold, 80-fold, 90-fold, 100-fold or even more compared with the reference value is considered as "increased" expression level. On the other hand, a decrease in expression levels below the reference value of at least 0.9-fold, 0.75-fold, 0.2-fold, 0.1-fold, 0.05-fold, 0.025-fold, 0.02-fold, 0.01-fold, 0.005-fold or even less compared with reference value is considered as "decreased" expression level.

Thus, according to the diagnostic method of the invention, if there is an increase in the expression level of miR-9 in a sample of a subject to be diagnosed with respect to the reference value, then said subject is diagnosed with a fibrotic disease.

The person skilled in the art will understand that the prediction may not be correct for 100% of patients under study. However, the expression requires that the prediction method provides correct results for a statistically significant portion of patients. Determination whether the method of the invention provides statistically significant predictions can be carried out by using standard statistical techniques such as the determination of confidence intervals, determination of p value, Student's t-test, Mann-Whitney test. Suitable confidence intervals are at least 50%, at least 60%, at least 70%, at least 80%, at least 90%, at least 95%. p values are, preferably, 0.05, 0.01, 0.005.

The invention is detailed below by means of the following examples which are merely illustrative and by no means limiting the scope of the invention.

### EXAMPLE 1. miR-9 modulates fibrogenic transformation of pulmonary fibroblasts in vitro

### Hydrogen peroxide (H₂O₂) and TGF-β1 induce miR-9 expression

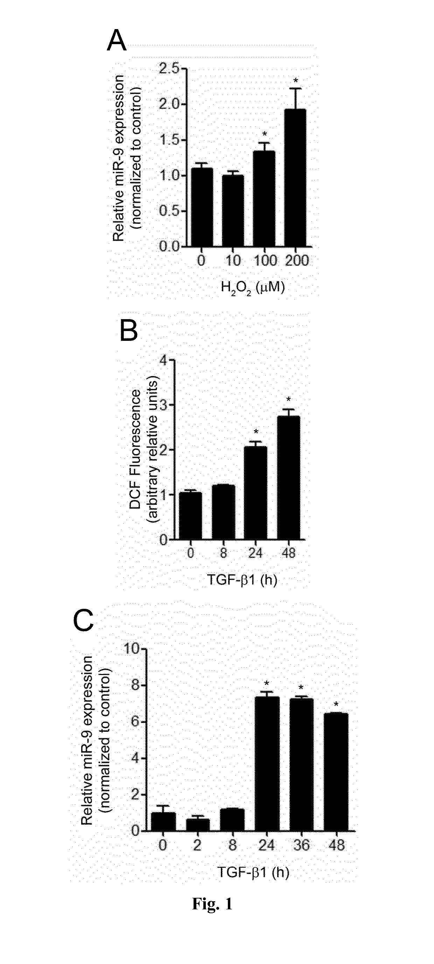

The inventors showed that H₂O₂ induced miR-9 expression in human lung fibroblasts (HFL-1) (see Figure 1A). Additionally, TGF-β1 induced ROS production, measured by flow cytometry using DCHF-DA probe (DCF) (Figure 1B) and upregulated miR-9 expression in HFL-1 (Figure 1C).

### miR-9 directly binds to the NOX4 and TGFBRII 3'UTRs and regulates their expression

*In silico* analysis of miR-9 targets using the computational algorithm Targetscan, predicted that NOX4 and TGFBRII, implicated both in the pathogenesis of organ fibrosis, are miR-9 targets.

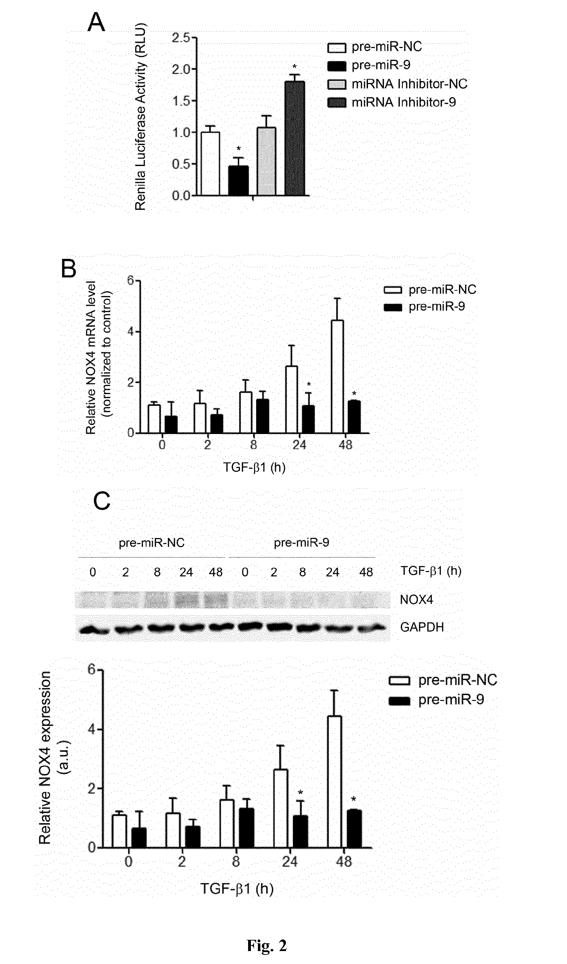

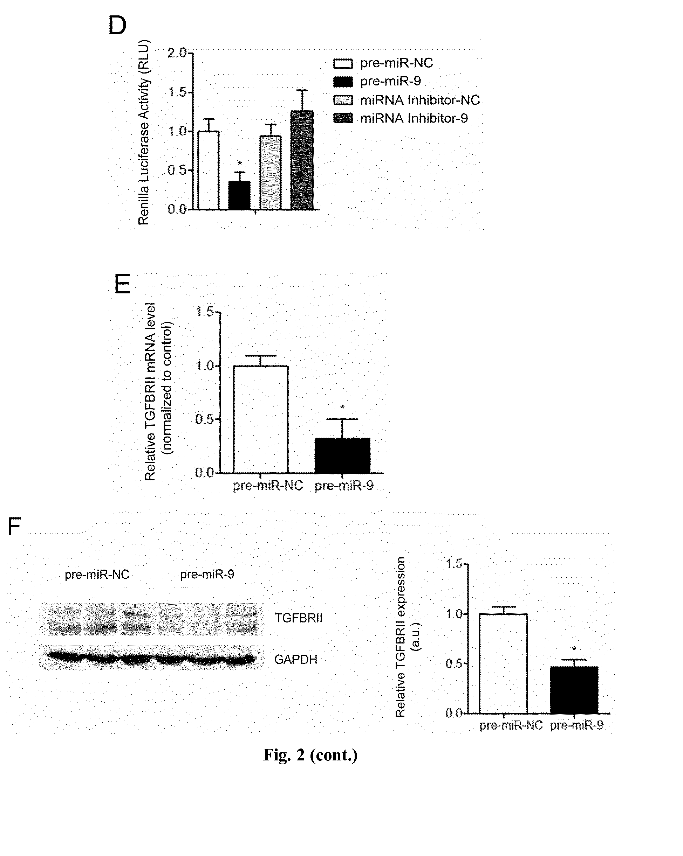

The inventors showed that miR-9 binds directly to the human 3'UTR of NOX4 and TGFBRII genes, detected by a decrease in the luminiscence of a luciferase reporter plasmid (Figure 2, A and D). Increasing miR-9 levels by transfection of miR-9 precursors decreased TGFBRII- and TGF-β1-NOX4-induced expression, at the mRNA (Figure 2, B and E) and protein levels (Figure 2, C and F).

### miR-9 overexpression attenuates TGF-β1-dependent transformation of fibroblasts into myofibroblasts

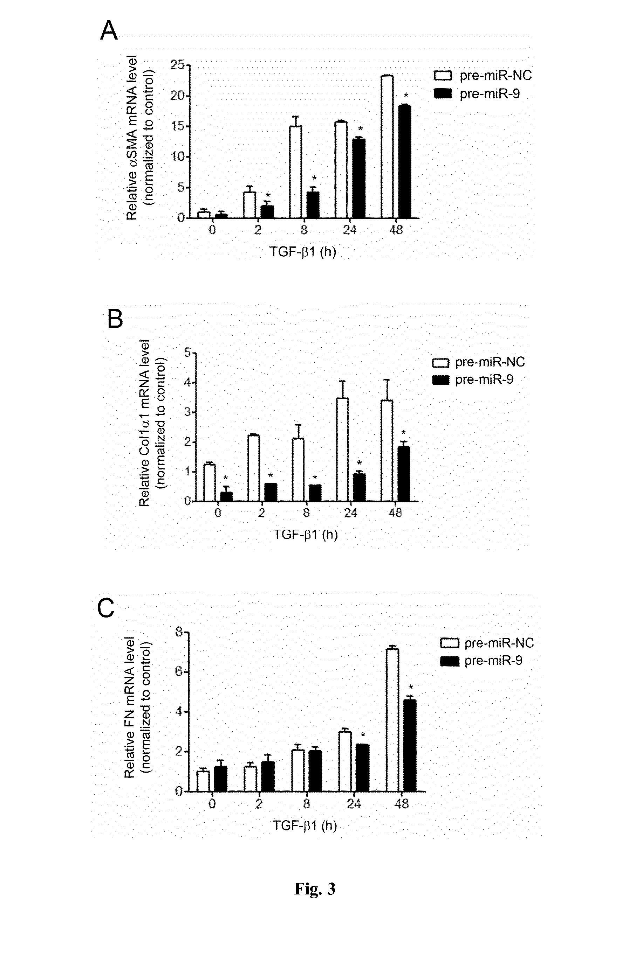

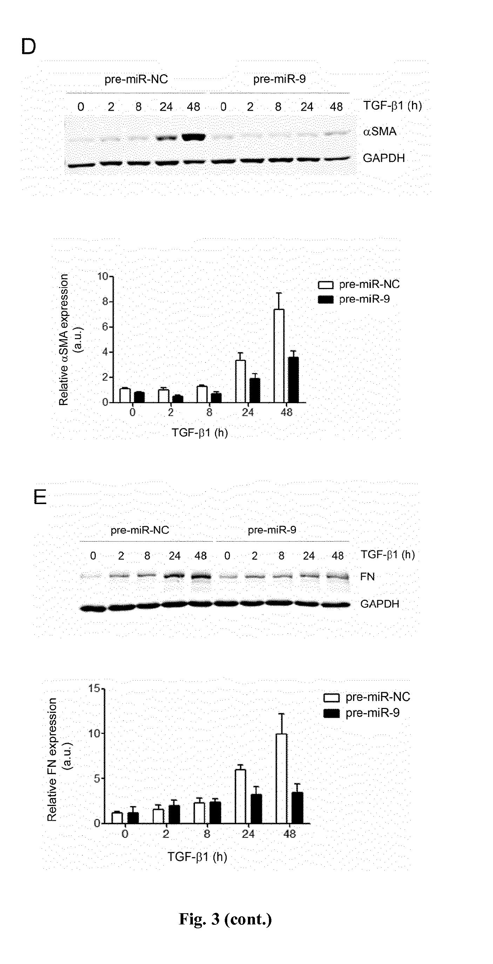

Increasing miR-9 levels in pulmonary fibroblasts delayed TGF-β1-induced transcription of α-smooth muscle actin (α-SMA), collagen 1α1 (Col 1α1) and fibronectin (FN) (Figure 3, A-C). Similarly, up-regulation of miR-9 decreased α-SMA and FN protein levels (Figure 3, D, E, and data not shown).

These data suggest that miR-9 plays a significant abrogatory role in the TGF-β1 mediated transformation of fibroblasts into fibrogenic myofibroblasts.

### miR-9 overexpression regulates the profibrogenic activities of TGF-β

The inventors analyzed whether the miR-9 antifibrogenic effect could be mediated through the regulation of TGF-β1-mediated signaling pathway.

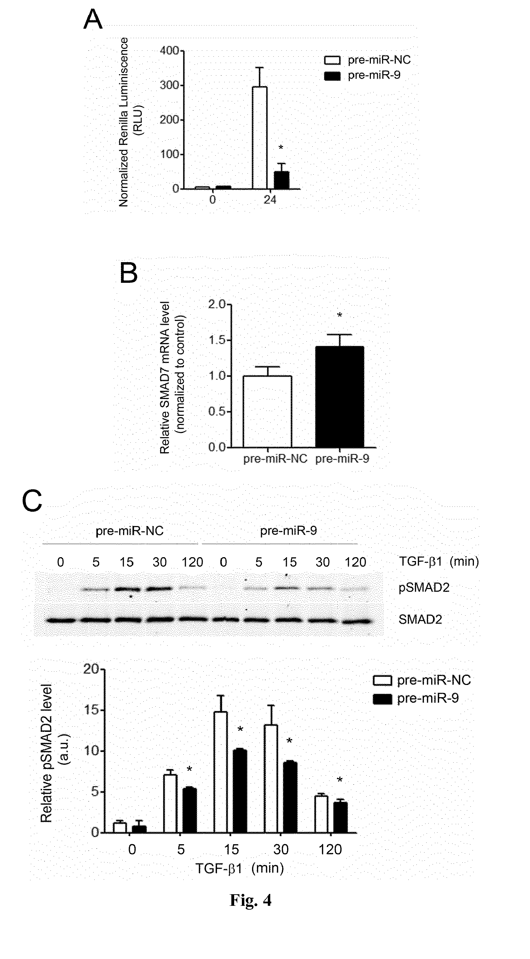

Consistent with this hypothesis, increasing miR-9 levels reduced Smad2/3 signaling, reflected in the decrease in the luminiscence of the CAGAluc luciferase reporter plasmid (Figure 4A), increased Smad7 expression (Figure 4B) and attenuated Smad2 phosphorylation (Figure 4C) in response to TGF-β1 stimulation in pulmonary fibroblasts.

The inventors analysed then the role of oxidative stress in the development and progression of human idiopathic pulmonary fibrosis (IPF). They found that both treatments, oxidative stress and TGF- β1, induced miR-9 expression in pulmonary fibroblasts. Increasing miR-9 levels significantly reduced TGFBRII and TGF- β1-NOX4 induced expression. This effect correlated with a decrease in TGF-β1-mediated signaling. Up-regulation of miR-9 levels attenuated TGF-β-induced transformation of lung fibroblasts into myofibroblasts. These contractile fibroblasts are believed to be the main source of abnormal extracellular matrix production in organ fibrosis. Thus, the inventors concluded that miR-9 downregulates the profibrogenic activities of TGF- β1 *in vitro.* Thus, the inventors analysed next the role of miR-9 in the pathogenesis and prevention of lung fibrosis *in vivo,* in order to establish its potential utility for the treatment of IPF.

### EXAMPLE 2. miR-9 modulates fibrogenic transformation of pulmonary fibroblasts in vivo

The lentiviral expression vector used by the inventors was the mouse pre-microRNA-9-1 from System Biosciences (Catalog No. MMIR-9-1-PA-1, for mouse miRNA precursor under accession number MI0000720, with sequence CGGGGUUGGUUGUUAUCUUUGGUUAUCUAGCUGUAUGAGUGGUGUGGAG UCUUCAUAAAGCUAGAUAACCGAAAGUAAAAAUAACCCCA (SEQ ID NO:5). The mmu-miR-9-1 pre-microRNA construct is cloned into a human immunodeficiency virus (HIV) lentiviral vector that has the miRNA precursor under the control of a CMV promoter, this robust strong viral promoter ensures a high level of expression from a single copy of integrated miRNA construct. The vector contains copGFP and a puromycin resistant gene driven by the human EF1α promoter. The copGFP marker is a novel natural green monomeric GFP-like protein from copepod (*Pontellina* sp.). The copGFP protein is a non-toxic, non-aggregating protein with fast protein maturation, high stability at a wide range of pH (pH 4-12), and does not require any additional cofactors or substrates. copGFP protein has very bright fluorescence that exceeds at least 1.3 times the brightness of EGFP, the widely used *Aequorea victoria* GFP mutant. Due to its exceptional properties, copGFP is an excellent fluorescent marker which can be used instead of EGFP for monitoring delivery of lentivector constructs into cells. The construct consists of a stem loop structure with approximately 100-300 base pairs of upstream and downstream flanking genomic sequence. The native structure ensures interaction with endogenous RNA processing machinery and regulatory partners, leading to the properly cleaved microRNAs. The lentivector was used to express mmu-miR-9-1 by transduction following viral packaging using pSAPAX2 and pMD2A plasmids.

Mice (n=4 in each group) received lentimiR scramble (lenti-SC) or lentimiR-9 (1x10⁶ IFU in 40 µl saline serum) by oropharyngeal administration 4 days before bleomycin instillation (1.5 U/kg body weight in 40 µl saline serum) or saline serum. After 14 days of bleomycin administration, mice were sacrificed and total RNA from lungs was isolated.

Pulmonary fibrosis, as assessed on day 0 and 14 after bleomycin administration, was determined in mice given lentimiR-SC or lentimiR-9. Hematoxylin and eosin (H&E) staining and Masson's trichrome blue staining was performed for collagen. α-SMA expression was determined by inmunohistochemistry using anti-α-SMA antibody.

### Increasing miR-9 expression prevents bleomycin-induced lung fibrosis in mice

After 14 days of bleomycin administration, mice were sacrificed and total RNA from lungs was isolated, and real-time PCR was performed to determine mRNA levels of α-SMA, Col 1α1 and FN in each group (Figure 5A). Increasing miR-9 levels in the lungs reduced the elevated expression of ECM proteins, such as Col 1α1 and FN at the mRNA level that occur in the lungs after bleomycin administration. The lenti-SC had no effects on bleomycin-induced lung fibrosis. Even more, α-SMA expression was not increased in bleomycin-treated lungs from mice given lentimiR-9, suggesting not only that overexpression of miR-9 decreased experimental pulmonary fibrosis but also that miR-9 played a significant abrogatory role in the induction of myofibroblasts differentiation.

Hematoxylin/eosin staining shoed a dramatic attenuation of bleomycin-induced lung fibrosis in mice pretreated with lentimiR-9 (Figure 5B, first line). This finding was confirmed by Masson's trichrome staining, which highlighted collagen deposition (Figure 5B, second line). Likewise, pretreatment with lentimiR-9 prevented the accumulation of myofibroblasts in bleomycin-treated lungs, as demonstrated by inmunohistochemistry staining with α-SMA antibody (Figure 5B, third line). Collagen (Figure 5C) and myofibriblasts (Figure 5D) were quantified in mice treated with lenti-miR-9 and control mice.

### miR-9 targets NOX4 bleomycin-induced and TGFBR2 in vivo

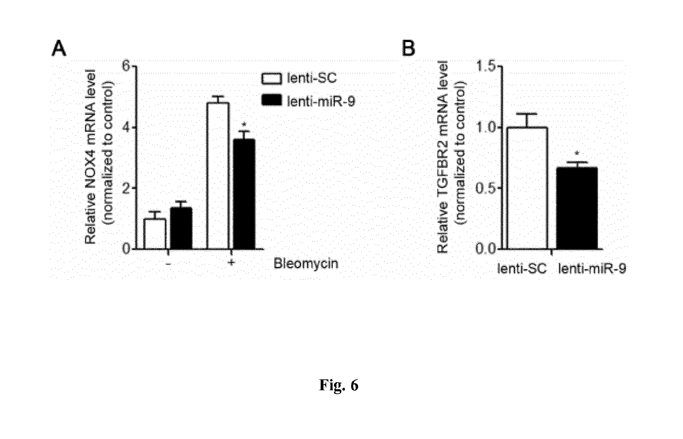

After 14 days of bleomycin administration, mice were sacrificed and total RNA from lungs was isolated. Real-time PCR was performed to determine mRNA levels of NOX4 in each group (Figure 6A). Increasing miR-9 levels in the lungs reduced the elevated expression of NOX4 at the mRNA level that occurs in the lungs after bleomycin administration. The lenti-SC had no effects on bleomycin-induced lung fibrosis.

After 4 days of lentivirus administration, mice were sacrificed and total RNA from lungs was isolated, and real-time PCR was performed to determine mRNA levels of TFBR2 in each group, showing that increasing miR-9 levels in the lungs reduced the expression of TGFBR2 at the mRNA level (Figure 6B).

### EXAMPLE 3. miR-9 is up-regulated in lungs from bleomycin-treated mice and from patients with idiopathic pulmonary fibrosis (IPF)

Total RNA was isolated from lungs harvested at the indicated time points after oropharingeal bleomycin administration (1.5U/kg body weight in 40 µl saline serum). Real-time PCR was performed to determine the levels of miR-9.

Lung sections were prepared from mouse lungs harvested at day 0 and 14 after bleomycin instillation. *In situ* hybridization was performed to determine the localization of miR-9.

miR-9 levels were determined in histologically normal lungs (n=3) and in lungs from patients with IPF (n=7). Bleomycin administration resulted in increased miR-9 expression over time (Figure 7A). miR-9 expression was higher in lungs from mice treated with bleomycin than in lungs from control mice (Figure 7B).

Additinally, lung samples from IPF patients showed increased miR-9 expression when compared to control subjects (Figure 7C).

### EXAMPLE 4. Increased miR-9 expression prevents epithelial-mesenchymal transition (EMT) of mesenchymal cells in the peritoneal fibrosis

The inventors found that TGF-β1 induced miR-9 expression in omentum derived mesenchymal cells (MCs).

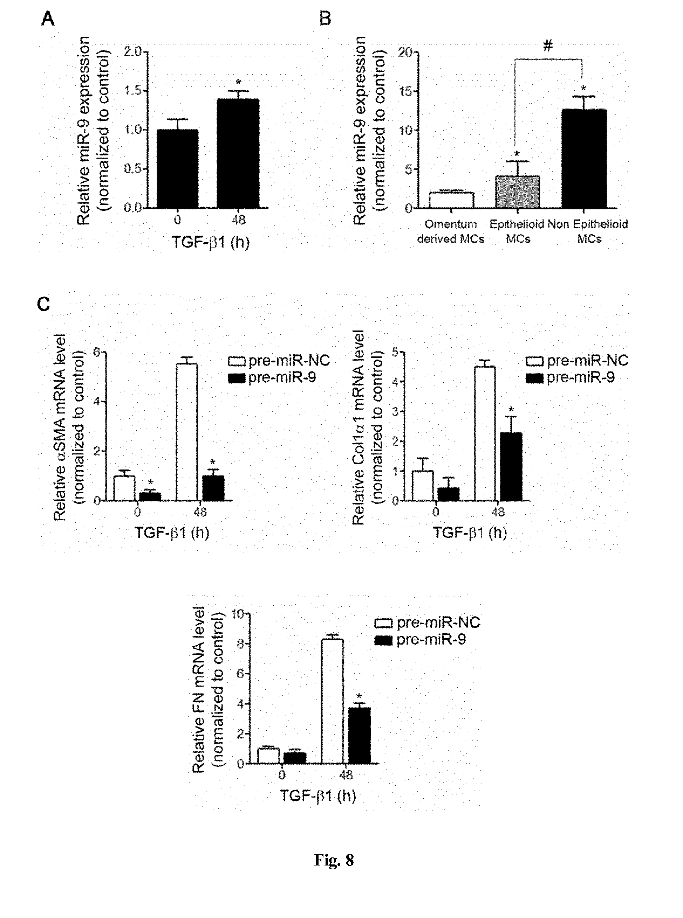

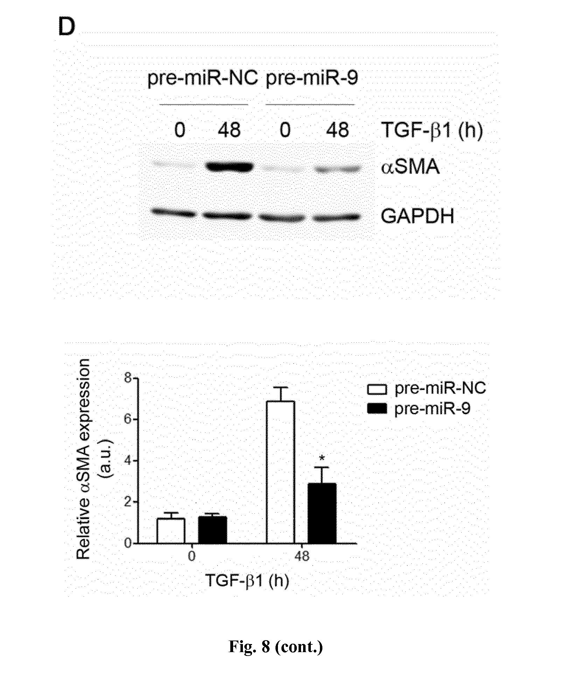

miR-9 was upregulated in non-epithelioid MCs derived from patients undergoing peritoneal dialysis compared to epithelioid MCs (Figure 8A). Additionally, MCs isolated from effluents in dialysis fluid of patients undergoing continuous peritoneal dialysis show increased expression of miR-9 compared with MCs from omentum (Figure 8B). Overexpression of miR-9 in MCs delayed TGF-β1-induced transcription of α-SMA, collagen 1α1 (Col 1α1) and fibronectin (FN) (Figure 8C). Similarly, up-regulation of miR-9 decreased α-SMA protein levels (Figure 8D).

These data suggested that miR-9 plays a significant role in the TGF-β1 mediated transformation of MCs into fibrogenic myofibroblasts, indicating a protective role for miR-9 in the initiation and progression of peritoneal fibrosis (PF).

### EXAMPLE 5. Increased miR-9 expression regulates the profibrogenic activity of TGF-β in human dermal fibroblasts

miR-9 expression in human dermal fibroblasts was measured by RT-qPCR. *In silico* analysis of miR-9 targets was performed using the computational algorithm Targetscan.

### TGF-β1 induced miR-9 expression in human dermal fibroblasts

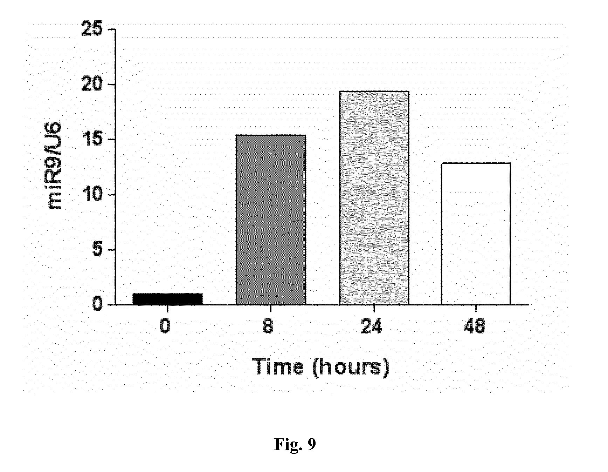

The inventors confirmed that TGF-β1 induced miR-9 expression also in human dermal fibroblasts (Figure 9).

### miR-9 regulated TGFBRII expression in human dermal fibroblasts

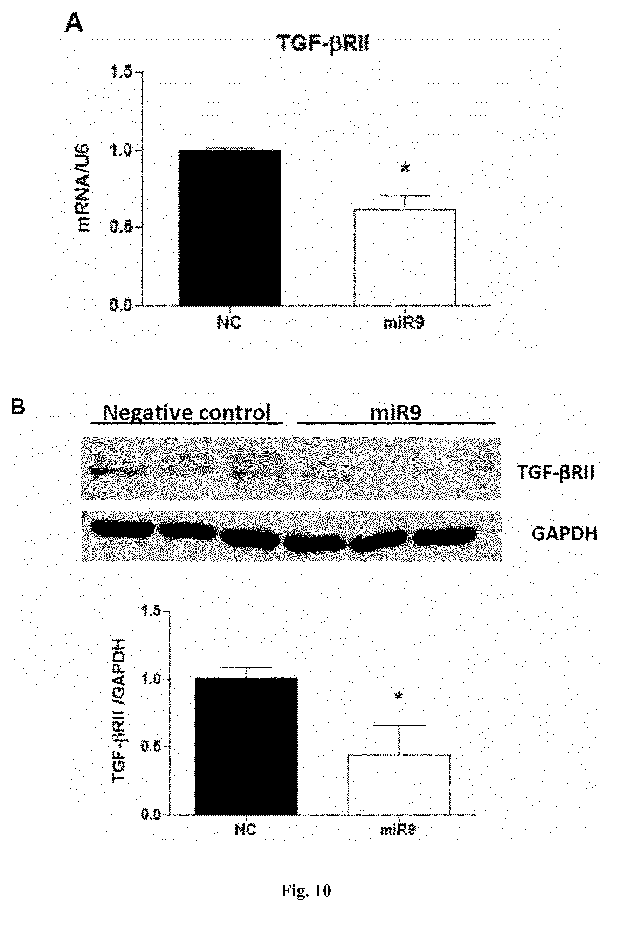

The *in silico* analysis of miR-9 targets predicted TGFBRII as a potential target of miR-9. Confirming this analysis, the inventors found that when miR-9 levels were increased by cell transfection of miR-9 precursors, TGFBRII was decreased at the mRNA (Figure 10A) and protein levels (Figure 10B).

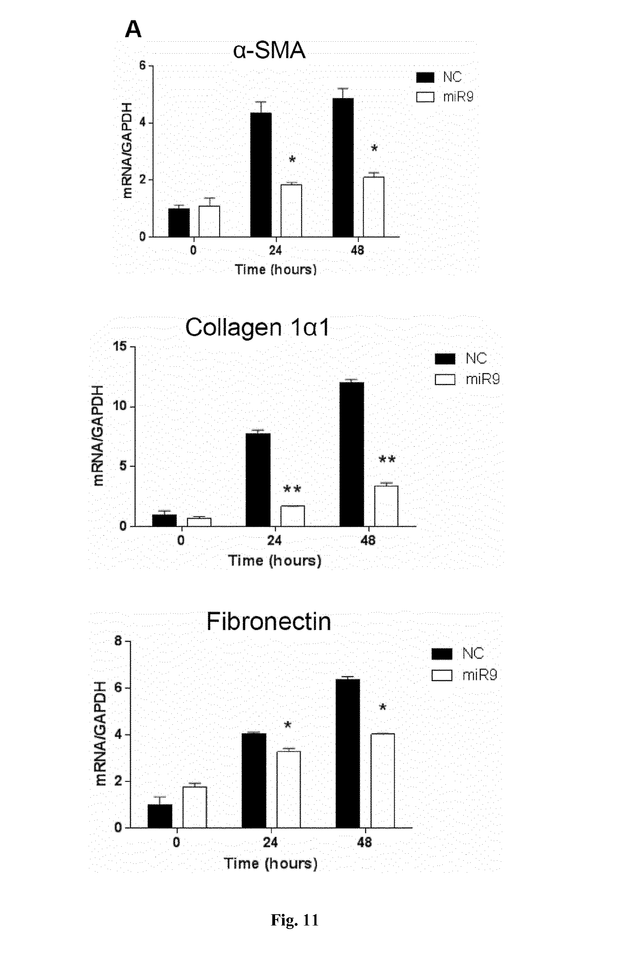

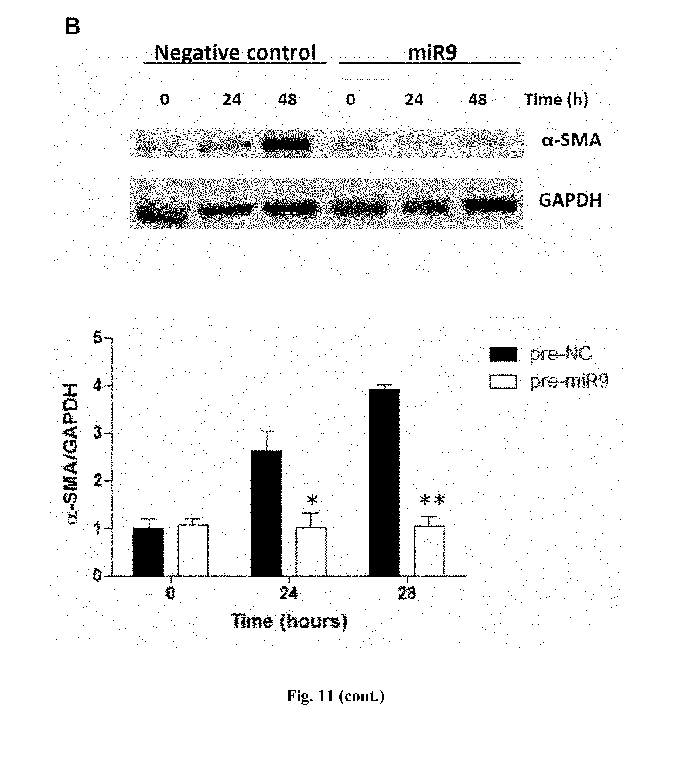

Similarly, increasing miR-9 levels in human dermal fibroblasts delayed TGF-β1-induced transcription of α-SMA, collagen 1α1 (Col 1α1) and fibronectin (FN) (Figure 11A). Up-regulation of miR-9 decreased α-SMA and FN protein levels as well (Figure 11B).

Thus, these data indicated that miR-9 plays a significant role in the TGF-β1 mediated transformation of fibroblasts into fibrogenic myofibroblasts, pointing out a protective role of miR-9 in the initiation and progression of skin fibrosis.

### miR-9 overexpression regulated the profibrogenic activities of TGF-β

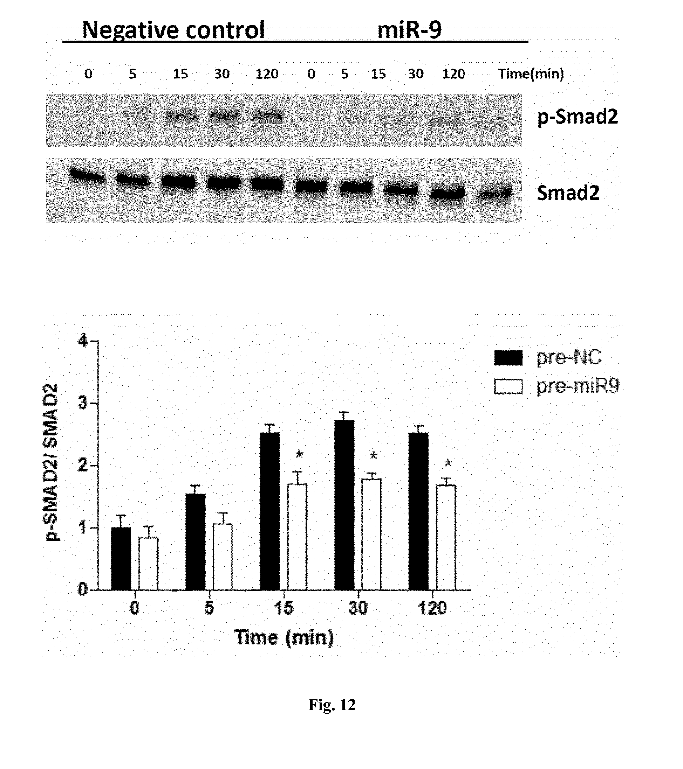

The inventors analysed whether the miR-9 antifibrogenic effect could be mediated through the regulation of TGF-β1-mediated signaling pathway. Consistent with this hypothesis, increasing miR-9 levels reduced Smad2 signaling, reflected in the attenuation of Smad2 phosphorylation in response to TGF-β1 stimulation in dermal fibroblasts (Figure 12).

## Claims

1. An agent selected from the group consisting of miR-9 (accession number MIMAT0000441 according to miRBase database, SEQ ID NO:1), a miR-9 variant, an agent inducing the expression of miR-9 and an agent inducing the expression of a variant of miR-9, for use in the prevention and/or treatment of a fibrotic disease in a subject.

2. The agent for use according to claim 1, wherein the agent inducing the expression of miR-9 is selected from the group consisting of a pre-miR-9, a polynucleotide encoding miR-9 and a polynucleotide encoding pre-miR-9.

3. The agent for use according to claim 2 wherein the polynucleotide is provided within a vector.

4. The agent for use according to claim 2 wherein the vector is a lentiviral vector.

5. The agent for use according to any of claims 2 to 4, wherein the pre-miR-9 is selected from the group consisting of hsa-mir-9-1 (accession number MI0000466 according to miRBase database, SEQ ID NO:2), hsa-mir-9-2 (accession number MI0000467 according to miRBase database, SEQ ID NO:3) and hsa-mir-9-3 (accession number MI0000468 according to miRBase database, SEQ ID NO:4).

6. The agent for use according to any claims 1 to 5, wherein the fibrotic disease is selected from the group consisting of idiopathic pulmonary fibrosis, peritoneal fibrosis and skin fibrosis.

7. The agent for use according to claim 6, wherein the fibrotic disease is idiopathic pulmonary fibrosis.

8. The agent for use according to the any of claims 1 to 7, wherein the subject is a human.

9. The agent for use according to any of claims 1 to 8 wherein the agent is administered oropharingeally.

10. A method for diagnosing a fibrotic disease in a subject comprising
(i) determining the level of expression of miR-9 in a sample from said subject, and
(ii) comparing the level of expression in (i) to a reference value,
wherein if the expression level of miR-9 is increased with respect to the reference value, then the subject is diagnosed with a fibrotic disease.

11. A method according to claim 10, wherein the reference value is the level of expression of miR-9 determined in a sample from a healthy subject.

12. The method according to claims 10 or 11, wherein the sample is a lung tissue sample.

13. The method according to any claims 10 to 12, wherein the fibrotic disease is selected from the group consisting of idiopathic pulmonary fibrosis, peritoneal fibrosis and skin fibrosis.

14. The method according to claim 13, wherein the fibrotic disease is idiopathic pulmonary fibrosis.

15. The method according to any of claims 10 to 14, wherein the subject is a human.
